# EUROPEAN PATENT SPECIFICATION

(11) **EP 2 797 494 B1**
(45) Date of publication and mention of the grant of the patent: **15.02.2017**
(21) Application number: 12864822.7
(22) Date of filing: 28.12.2012
(51) Int. Cl.: A61B 3/14, A61B 1/32

(54) **OPHTHALMIC SYSTEMS FOR MONITORING EYES**
OPHTHALMOLOGISCHE SYSTEME ZUR ÜBERWACHUNG DER AUGEN
SYSTÈMES OPHTALMIQUES POUR SURVEILLER LES YEUX

(30) Priority: 29.12.2011 US 201161581591 P
(43) Date of publication of application: 05.11.2014
(73) Proprietor: Neuroptics, Inc., Irvine, CA 92612 (US)
(72) Inventor: SIMINOU, Kamran, Newport Coast, CA 92657 (US)
(74) Representative: Viering, Jentschura & Partner mbB Patent- und Rechtsanwälte
(86) International application number: PCT/US2012/071887
(87) International publication number: WO 2013/106203

(56) References cited:
- WO-A2-2004/054487
- CN-U- 2 033 668
- US-A- 3 651 689
- US-A- 5 582 608
- US-A- 5 762 606
- US-A1- 2002 002 369
- US-A1- 2003 095 781
- US-A1- 2008 284 979
- US-A1- 2009 213 329
- US-B1- 6 267 752
- US-B1- 6 535 223

## Description

### Background

This application claims the benefit of priority to U.S. Provisional Patent Application No. 61/581,591, filed December 29, 2011.

The invention relates to an automated ophthalmic monitoring system according to the preamble of claim 1. Hence, this disclosure relates to instruments, systems, and methods of monitoring the eyes of subjects, such as while they are in a coma, unconscious, paralyzed, sedated, suffering from concussions or mild to severe traumatic brain injury or in other such conditions whereby they are unable to voluntarily open and/or close their eyelids.

An automated ophthalmic monitoring system of the above-mentioned type is known, e.g., from US 2009/213329 A1. A further automated ophthalmic monitoring system is known, e.g., from US 3 651 689 A. The continuous monitoring of the ocular health and/or neurological status of patients, particularly their brain function and health during periods of trauma, unconsciouness, sedation, and/or other such conditions is an important aspect of patient care. Having an indication of the ocular health and/or neurological status of a patient can be useful to prevent harm to the patient during, e.g., surgical procedures or periods of sedation, or can help assess the neurological status and/or the likelihood of morbidity or mortality during, before, or after administration of a treatment, such as CPR, or in a comatose, unconscious or traumatized patients.

One example of this is the potential for damage to eyelids, cornea, peri-orbital soft tissues, and vision (including permanent blindness) of a sedated, anesthetized, or other affected patient during surgical procedures, such as wherein the patient is in a prone or lateral position, for instance, during spinal surgery. The risk of blindness and damage may be associated with many factors, including (1) prolonged prone positioning (which produces dependent facial, ocular, and peri-orbital venous congestion and edema), (2) Trendelenberg (head-down) positioning, (3) sources of increased extra-ocular pressure causing increased intra-ocular pressure, (4) baseline intrinsic increased intra-ocular pressure (potentially causing optic-nerve damage), (5) general systemic hypotension, (6) low hemoglobin oxygen saturation levels, (7) increased intra-abdominal pressure, particularly during prone positioning, (8) central retinal artery thrombosis, (9) pre-existing sub-clinical retinal disease or retinal vascular disease, (10) direct mechanical trauma to the peri-orbital tissues, and (11) optic nerve ischemia from other causes. Monitoring pupils during surgery for relative afferent pupillary defect (RAPD) could alert physicians to optic nerve ischemia or any one of the problems set forth above. However, at the present time there is no adequate means for consistent monitoring, and thus, there is a need for automated means for performing such monitoring of RAPD.

Another example that highlights the importance of having real-time information about the neurological status, such as status of brain health, of a patient is when patients are unconscious and in need of CPR. Pupil reaction to light during CPR is a standard method of assessing the neurological health of the individual undergoing CPR. Studies have shown that dynamic changes of pupil diameter and reactions to light during cardiac arrest and resuscitation are correlated with coronary perfusion pressure, and are predictive of the likelihood that spontaneous circulation and cerebral function would be restored. Zhao, Danhong, Pupil diameter and light reaction during cardiac arrest and resuscitation, Critial Care Medicine, April 2001, V.29, Issue 4, pp 825-828.

One problem associated with monitoring pupils in subjects while they are in a coma, unconscious, paralyzed, sedated, or the like is that they are often unable to voluntarily open and/or close their eyelids. However, keeping eyelids open for long periods of time while performing CPR, medical treatments, or other surgical procedures will dry out the subject's corneas and may cause damage to them.

It is also very important to monitor on an ongoing basis the neurological status of comatose or incapacitated patients who are suffering from a concussion, stroke, or other brain injury between the spectrum of mild brain injury to severe brain injury. Currently, medical practitioners require multiple ways to monitor and assess the neurological status of patients who have experienced brain injury or brain trauma. This is due to the complexity of intracranial dynamics and the complex physiology of brain injury. One of the physiological characteristics that medical practitioners monitor is intra-cranial pressure (ICP). Patients with a Glasgow Coma Scale (GCS) score between 3 and 8 are candidates for ICP monitoring (Bader & Littlejohns, 2004). ICP is monitored in order to assess a patient's neurological status. Some indications for ICP monitoring include aneurismal subarachnoid hemorrhage, brain tumor, decompensated hydrocephalus, cerebral hypoxia or anoxia producing edema, traumatic brain injury, and Reyes syndrome, among others (id). Currently, ICP is monitored using an invasive procedure that requires placement of a catheter into the brain and connecting it to a pressure transducer. A significant disadvantage to the current method of monitoring ICP is that the procedure is invasive and potentially dangerous. Nonetheless, it is an important procedure, because it provides the practitioner with a means to assess the neurological status of the patient.

Neurological deterioration and brain injury can also be assessed by clinicians manually using a pupil gauge and a flashlight. This is a non-invasive procedure, but is imprecise and does not always lead to the appropriate diagnoses and provision of appropriate care.

There is currently no automated manner in which to noninvasively continuously monitor the eyes of a subject for neurological status of patients who are believed to have suffered brain injury and are in a coma or are otherwise incapacitated or who are undergoing a medical procedure, such as a surgical procedure in which they are sedated.
Thus, there is a need for instruments, such as automated instruments and systems that can open and/or close eyelids of subjects who cannot voluntarily open and close their eyelids, and can capture and analyze data associated with the their eyes, such as a pupils' dynamic response to stimulus, such as light. There is a further need for such instruments and systems that can perform the above tasks automatically without inordinate attention of a physician or healthcare professional while a treatment, CPR, or surgical procedure is taking place, or when the patient is in a coma or is otherwise incapacitated.

### Summary

The invention provides an automated ophthalmic monitoring system according to claim 1.
Further embodiments of the invention are described in the dependent claims.

### Brief Description of the Drawings

Figure 1 is a three-dimensional view of an eyelid retractor.
Figure 2 is a three-dimensional view taken from the bottom of the eyelid retractor of Figure 1.
Figure 3 is an illustration of the eyelid retractor of Figure 1 when adhered to the head of a subject.
Figure 4 is a three-dimensional view of a retractor cable assembly.
Figure 5 is a three-dimensional view of the retractor cable assembly of Figure 4 when coupled to the eyelid retractor of Figure 1.
Figure 6 is an illustration of a pair of eyelid retractors when adhered to the head of a subject over each of the subject's eyes.
Figure 7 is a three-dimensional view taken from the bottom of a pupilometer assembly.
Figure 8 is a three-dimensional view taken from the top of the pupilometer assembly of Figure 7.
Figure 9 is a three-dimensional view taken from the top of the pupilometer assembly of Figure 7 in an extended state.
Figure 10 is an illustration of the pupilometer of Figure 7 mounted on the eyelid retractor of Figure 1.
Figure 11 is an illustration of a pupil monitoring system including the pupilometer of Figure 7, the eyelid retractor of Figure 1, and an automated control unit for controlling the pupilometer and the eyelid retractor.
Figure 12 is a three-dimensional view of a surgical face mask.
Figure 13 is a three-dimensional view of the interior surface of the face mask depicted in Figure 12.
Figure 14 is an illustration of the surgical face mask of Figure 12 as worn by a subject.
Figure 15 is a top view of the surgical face mask of Figure 12 as worn by a subject.
Figure 16 is a three-dimensional view of a pupilometer with binocular imaging sensors or cameras.
Figure 17 is a three-dimensional view taken of the interior surface of an optional surgical face mask retention casing.
Figure 18 is an illustration of an ocular monitoring system including the surgical face mask of Figure 12, the surgical face mask retention casing of Figure 17, and the pupilometer of Fig. 16, in communication with an automated ocular monitoring control unit during a surgical procedure.
Figure 19 is an illustration of a subject wearing an ocular monitoring assembly including the surgical face mask of Figure 12, optional face mask retention casing of Figure 17, and pupilometer of Figure 16.
Figure 20 is a schematic diagram of another embodiment of an eyelid retractor.
Figure 21 is aperspective view of an eyelid retractor in accordance with the embodiment depicted in the schematic diagram of Figure 20. of
Figure 22 is another perspective view of the eyelid retractor depicted in Figure 21.
Figure 23 is a perspective exploded view of an eyelid retractor and connectorable pupilometer.
Figure 24 is a perspective view of the eyelid rectractor and pupilometer of depicted in Figure 23 in a connected configuration.
Figures 25 a and 25b are flowcharts depicting the steps of a program used to control the operation of a pupilometer and eyelid retractor.

### Detailed Description

As set forth above, there is a need for instruments, such as automated instruments and systems that can open and/or close the eyelids of a subject, such as for one who cannot voluntarily open and/or close their eyelids. In addition there is a further need for automated instruments, such as a pupilometer (or other ophthalmic instruments used to monitor the eye or eyes of a subject, such as tonometers, retinascopes, slit lamp bio microscopes, ophthalmoscopes, and keratometers), and analyze data associated with the state of a subject's eye(s), or more specifically, such as the pupils' dynamic response to stimulus, such as light. Accordingly, the instruments and systems of the disclosure are configured such that they can perform the above tasks automatically, such as without inordinate attention of a physician or healthcare professional handling or manipulation while a treatment, CPR, surgical procedure or when the patient is in a coma or is otherwise incapacitated.

The pupilometers described herein contain associated control units and software for analyzing the activity of a subject's pupil(s) and providing an output or signals indicative of various neurological disorders or neurological conditions, including those associated with optic nerve disease, or damage caused to the optic nerve while a procedure is taking place, such as damage from ischemia during monitoring of the pupil(s). Such pupilometers and their associated software and control units are described, for example, in U.S. Patent Nos. 8,235,526, 7,967,442, and U.S. Patent Application Serial Nos. 12/436,469 and 13/543,341.

Accordingly, in one embodiment, an automated eyelid retractor is provided. The eyelid retractor has a first component that is removably attachable to an eyelid. For example, it can be an eyelid patch with an adhesive backing on one end that is suited for adhesion to skin. Such adhesive backings are known in the art, and can be purchased from those who make adhesive materials, such as 3M®, which makes such an adhesive material. A second component is connected to the first component either directly or indirectly through a connector. The second component includes a housing that is removably attachable to the head of a subject, or to a fixed and stationary object, such as a part of bed or a stationary control unit near the subject's head. In one example, the second component can be attached to the head of the subject through a removable headband or belt applied to the subject's head. In another embodiment, the second component is attached to a bedpost. In yet another embodiment, the second component is attached to a stationary control unit. It can also have an adhesive backing on one of its sides and that side can be adhered to the skin on the subject's forehead and/or shaved head. Thus, the first component is attached to the eyelid of the subject while the second component is attached to the head of the subject or to some other stationary object. The two components are connected to one another directly or indirectly through a connector. The eyelid retractor also has a mechanism that controls the position or location of the first component relative to the position or location of the second component. The automated mechanism can retract the eyelid by moving the first component toward the second component, and can allow the eyelid to again close by allowing the first component to pull (or be pushed) away from the first component. The automated mechanism can include a microprocessor (or other electronics with logic capabilities, such as discrete logic) with a programmable memory that can be programmed to cause the mechanism to control the position of the first component relative to the second component in a predetermined sequential pattern. The automated mechanism can include electronics capable of receiving a signal from an external source that controls the automated mechanism, such as an external control unit with a wireless transmitter, while the automated mechanism contains a wireless receiver (alternatively the control unit can be hard-wired to the automated mechanism). The control unit can transmit a signal (such as, e.g., an electrical, mechanical, optical, or electromagnetic (wireless) signal) to the automated mechanism if they are hard-wired to each other. If they communicate with each other wirelessly, then the automated mechanism and the control unit with the microprocessor can communicate with each other through a wireless signal, such as a radio-frequency signal, an infra-red signal, a wireless USB signal, or a Bluetooth® signal. In any case, the mechanism is operable without direct physical manipulation by a medical practitioner, and can operate to move the first component so that the eyelid can be automatically opened or closed. The external control unit can be a remote control unit, a pupilometer, a desktop computer, a laptop computer, a smart telephone, a personal electronic device, a tablet computer, or any device with a programmable microprocessor that can be programmed to control the movement of the first component relative to the second component through operation and control of the automated mechanism.

In another embodiment, an automated ophthalmic monitoring system for monitoring the eyes of a subject is provided. The automated ophthalmic monitoring system includes one or more of an eyelid retractor, an ophthalmic instrument, such as, but not limited to, a pupilometer, a tonometer, a retinascope, a slit lamp bio microscope, an ophthalmoscope, or a keratometer, and an automated control unit. The eyelid retractor may have an attachment element that is configured for adhering the eyelid retractor to the skin of the subject. The eyelid retractor may additionally include a support member for supporting a pupilometer thereon.

Hence, in certain instances, the system includes an ophthalmic instrument, such as, but not limited to, a pupilometer, a tonometer, a retinascope, a slit lamp bio microscope, an ophthalmoscope, or a keratometer, that is coupled to the eyelid retractor. If the ophthalmic instrument is a pupilometer, it may have an imaging sensor or camera and a light emitter that can emit light in various forms, including in the form of a flash or amplitude modulated or adjusted light, so as to stimulate the pupil. The system may further include an automated control unit, which automated control unit is in communication with the eyelid retractor and/or the pupilometer. The automated control unit is configured for controlling the eyelid retractor and/or the ophthalmic instrument.

The instruments and systems of the disclosure are described in greater detail herein below with respect to the appended figures.

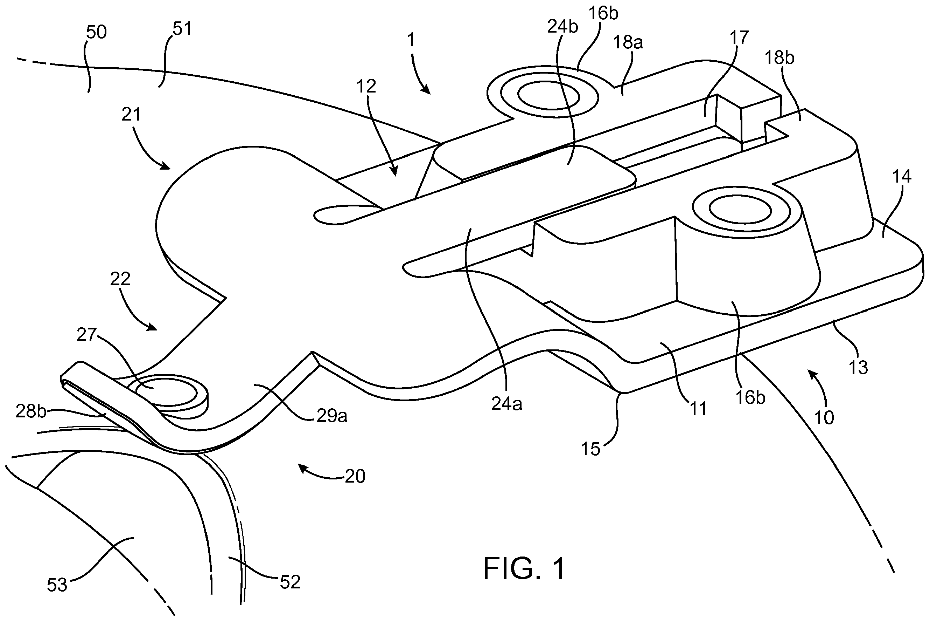

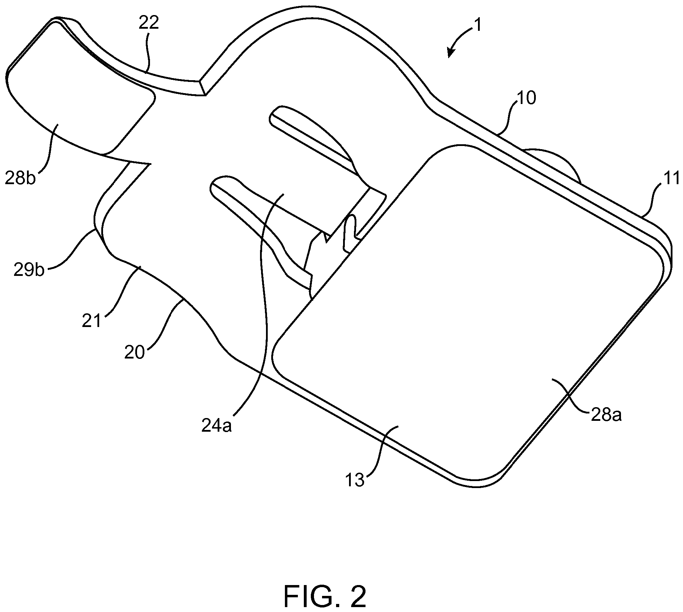

Accordingly, with respect to FIG. 1 an eyelid retractor 1 is provided. The eyelid retractor 1 has an anchoring portion 10 and a retraction portion 20. The anchoring portion 10 has a base member 11 that includes both top 12 and bottom 13 surfaces and further includes a proximal portion 14 having a proximal end and a distal portion 15 having a distal end. The anchoring portion 10 is at the proximal portion of the eyelid retractor 1. The bottom surface 13 of the anchoring portion 10 has an attachment interface 28a (see Fig. 2), such as an adhesive backing or suction cup(s), for adhering the anchoring portion 10 to a subject's forehead. The base member 11 may be curved so as to match the contours of the patient's forehead.

The eyelid retractor 1 also has a retractor portion 20 that may be contiguous with or otherwise capable of being coupled to the anchoring portion 10. In certain instances, the retractor portion 20 is associated with the anchoring portion 10 such that it extends distally from the anchoring portion 10. The retractor portion 20 includes a retraction member 21, which member may be a curved, flexible member. The retraction member 21 has an eyelid attachment or anchoring member 22, such as on its distal portion.

The eyelid attachment member 22 may be flat or curved (as shown in the figures) for attaching to an eyelid 53 of a patient 50 and thereby assisting in the opening and/or closing of the eyelid 53. The retractor portion 20, including the eyelid attachment member 22, has a top surface 29a and a bottom surface 29b (see Fig. 2) and extends distally from the anchoring portion 20. The bottom surface 29b of the eyelid attachment member 22 has an attachment interface 28b. The attachment interface 28b can have an adhesive, suction cup(s) or other detachably anchoring component or material to detachably attach the attachment member 22 to the eyelid 52 of the subject 50.

The anchoring portion 10 is moveably associated to the retraction portion 20. Hence, the retraction portion 20 is adapted for moving relative to the anchoring portion 10. This moveable association can have any suitable configuration so long as controllable movement is allowed between the anchoring portion 10 and the retraction portion 20. For instance, the anchoring portion 10 and retraction portion 20 may be separate individual members that are coupled in moveable orientation to one another, or they may be two portions of the same member that are configured so as to be moveable in relation to one another. In this embodiment, the anchoring portion 10 and retraction portion 20 are part of the same member, but are moveable in relation to one another.

Hence, in this instance, the anchoring portion 10 is configured for both anchoring the eyelid retractor 1 to the head 51 of the subject 50, and for assisting in the retraction of the retraction portion 20 and/or the opening and/or closing of the eyelid 52 of the eye 53. The retraction portion 20 is configured for both attaching to the eyelid 52 of the patient 50, and for moving in relation to the anchoring portion 10, which movement corresponds to the opening and/or closing of the eyelid 50. Accordingly, the anchoring portion 10 anchors the retraction portion 20 as it moves relative to the anchoring portion 10. It is to be noted that although a particular configuration for enabling the movement of the retraction portion 20 to the anchoring portion 10 is herein depicted any suitable configuration can be employed so as to achieve the desired opening and/or closing of the eyelid 52.

In this embodiment, the retraction portion 20 includes a retraction member 21, which retraction member 21 is comprised of a flexible material that is adapted for allowing the attachment member 22 to move toward and/or away from the anchoring portion 10. To facilitate the control of this movement, the retraction portion 20 includes a channel interface member 24a that translates within a channel 17 of the anchoring base member 11. The channel 17 includes a plurality of boundary members 18 a,b together which form the channel 17 within which the channel interface 24a translates. For instance, as a tension is applied to the channel interface member 24a, the flexible retraction member 21 flexes, the channel interface member 24a moves toward the proximal portion 14 of the base member 11, thereby causing the attachment member 22 to retract and consequently effectuating the movement of the attached eyelid 52, e.g., from a closed to an open position resulting in the exposing of the eye 53.

Conversely, as tension is released from and/or a force is applied to the channel interface member 24a, the flexible retraction member 21 returns to its rest position (and/or beyond its rest position, e.g., if a forced is applied thereto), moving the channel interface member 24a toward the distal portion 15 of the base member 11, thereby causing the attachment member 22 to return the eyelid 52 to its beginning position, which position may be a closed position. A further force may be applied to the channel interface member 24a so as to further assist in the movement of the component parts of the eyelid retractor 1 and thereby assisting in the closing of the eyelid 52 and the covering of the eye 53.

Accordingly, in certain aspects of the disclosure, an eyelid retractor having a proximal end and a distal end is provided. The eyelid retractor includes an anchoring portion and a retractor portion. The anchoring portion is provided on the proximal end of the eyelid retractor. The anchoring portion includes a top side and a bottom side, the bottom side includes an adhesive backing, suction cup(s) or other detachably anchoring component for detachably attaching the anchoring portion to a subject's forehead. The retractor portion extends distally from the anchoring portion. The retractor portion includes a retraction mechanism, and an eyelid attachment member that is positioned on the distal end of the eyelid retractor. In alternative embodiments, not shown, the anchoring portion can be secured to the head of a subject using a head band or a head belt that encircles the subject's head. In yet another embodiment, the anchoring portion is secured to a bed post, or another stationary object, such as a stationary control unit.

The eyelid attachment member includes a top side and a bottom side, wherein the the bottom side includes an adhesive backing, suction cup(s) or other detachably anchoring component for detachably attaching the anchoring portion to a subject's forehead. The eyelid attachment member extends distally form the retractor portion. The retractor portion is configured for pulling and/or pushing the eyelid anchoring portion in a proximal or distal direction, e.g., when the retraction mechanism retracts in the proximal direction, or extends in the distal direction. In certain instances, the retractor portion includes a flexible portion that is distal to or forms a distal part of the retraction mechanism.

For instance, the retraction mechanism may include a retraction arm that extends proximally, so as to from a channel interface member. The retraction arm may further include a connector or connection element that connects the retraction arm to a cable, such as a cable that is connected to a device, such as a motor, the operation of which effectuates the retraction and/or extension of the retraction mechanism. Hence, the retraction arm may be coupled to a device, such as a motor, that drives the retraction arm in both a proximal and a distal direction. In certain instances, the anchoring portion contains one or more connectors for connecting ophthalmic instruments to the eyelid retractor, and the top side of the eyelid attachment member may include a size calibration marker.

FIG. 2 provides a bottom-up perspective view of the eyelid retractor device of FIG. 1. The eyelid retractor device 1 includes an anchoring portion 10 and a retraction portion 20. The anchoring portion 10 includes a base member 11 having a bottom surface 13. The bottom surface 13 of the base member 11 includes an attachment interface 28a. The attachment interface 28a includes an adhesive, suction cup(s) or other detachably anchoring component or material to detachably attach the base member 11 to the skin of the subject 50. Thus, the attachment interface 28a is configured for coupling the base member 11 of the anchoring portion 20 with the forehead 51 of the patient 50.

The retraction portion 20 includes a retraction member 21 having both an attachment member 22 and a channel interface 24a. The attachment member 22 includes a bottom surface 29b having an attachment interface 28b associated therewith. The attachment interface 28b is configured for coupling the eyelid attachment member 22 with the eyelid 53 of the patient 50.

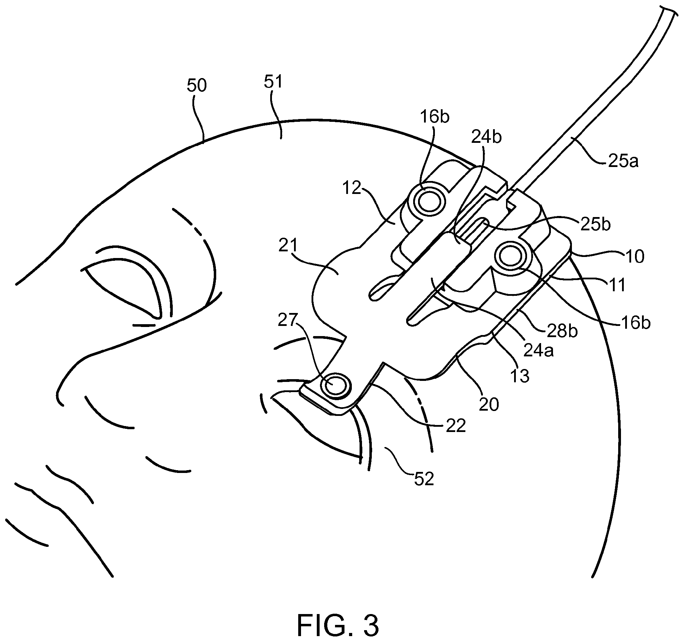

FIG. 3 provides an illustration of the eyelid retractor of FIG. 1 when adhered to the head of a subject. The retractor would be employed so as to control the opening and/or closing of an eyelid in a system, such as in an automated ophthalmic monitoring system for capturing and analyzing data associated with a subject's eye(s). In such an instance, the eyelid retractor may be configured for being coupled with an ophthalmic instrument, such as that disclosed herein. For example, the eyelid retractor 1 may include one or more support members 16 a,b that are configured for interacting with at least a portion of an ophthalmic instrument so as to support the ophthalmic instrument.

As can be seen with respect to FIG. 3, the eyelid retractor 1 is configured for being employed within a system for effectuating the opening and/or closing of an eyelid 52. The eyelid retractor 1 includes an anchoring portion 10 that comprises the proximal portion of the eyelid retractor 1, and a retractor portion 20 that comprises the distal portion of the eyelid retractor 1. As depicted the retractor portion 20 extends distally from the anchoring portion 10. The anchoring portion 10 includes a base member 11 having a top surface 12 and a bottom surface 13. The bottom surface 13 includes an attachment interface 28a. The attachment interface 28a includes an adhesive, suction cup(s) or other detachably anchoring component or material to detachably attach the base member 11 to the skin of the subject 50. The top surface 12 of the base member 11 may include a channel 17, bounded by opposing channel members 18 a,b, within which a retraction mechanism operates so as to control the retraction and/or extension of the retractor portion 20.

Accordingly, the eyelid retractor 1 includes a retraction mechanism, which retraction mechanism may include one or more of a channel interface member 24a having a connection interface 24b, a retraction member 21, and an eyelid attachment member 22. The channel interface 24a is configured for translating within the channel 17 and thereby moving the retraction member 21, which in turn moves the attachment member 22. The eyelid attachment member 22 is capable of engaging the eyelid 52 of the subject 50. For this purpose, the eyelid attachment member 22 includes a top surface 29a and a bottom surface 29b, wherein the bottom surface 29b includes an attachment interface 28b that detachably attaches the attachment member22 to the subject's eyelid 52. The attachment interface 28b can have an adhesive, suction cup(s) or other detachably anchoring component or material to detachably attach the attachment member 22 to the eyelid 52 of the subject 50.

The retraction portion 20 therefore is configured for moving in response to the retraction mechanism. For instance, the retraction mechanism may be operated so as to move, e.g., pulling, the eyelid 53 from a first position, e.g., a closed position, to a second position, e.g., an open position, and vice versa in response to the control asserted by the retraction mechanism. Alternatively, the retraction mechanism may be operated so as to move, e.g., push, the eyelid 53 from a first position, e.g., an open position, to a second position, e.g., a closed position in response to the control asserted by the retraction mechanism. These movements may be accomplished in such a manner that the attachment member 23 is moved in a proximal direction, such as when the retraction mechanism retracts the retraction member 21 in the proximal direction, or in a distal direction such as when the retraction mechanism extends the retraction member 21 in the distal direction.

The retraction mechanism may further include a retractor cable 25 that is connected at its proximal end to an automated control unit 40 as further described below. The retractor cable can be connected at its distal end to a connection interface 24b of a channel interface member 24a of the retraction portion 20. Hence, in certain embodiments, a retractor cable 25 is connected on its distal end to a connection interface 24b of the retraction mechanism and connected on its proximal end to the automated control unit 40, which can contain a device, such as a motor, for controlling the proximal and distal translation of the connection interface 24b via communication through the cable 25. Proximal and distal translation of the connection interface 24b drives retraction and/or extension of the retraction portion 20.

Accordingly, the control unit 40 may include a device, such as an electronic motor, that drives the retraction mechanism. For example, the device may function to assert a pulling force on to the connection interface 24b of the channel interface member 24a. This force may be translated via a wire 25c within cable 25 and communicated to the connection interface 24b via the connection element 25b. In response to this pulling force, the channel interface member 24a may translate within the channel 17, e.g., in a proximal direction, causing the retraction member 21 to flex and or otherwise move, bend, or retract, e.g., proximally, in response to the applied force. The retraction of the retraction member 21 in turn results in the proximal movement of the eyelid attachment member 22 and the opening of the eyelid 52.

It is to be noted that where the force applied is a pulling force, the retraction member 21 will retract, thus resulting in the moving of the eyelid 53 to an open position. However, where the force applied is a releasing and/or pushing force the retraction member 21 may return to its normal, at rest position, or be further extended (dependent on the nature of the restoring force), so as to move the eyelid 53 to a closed position.

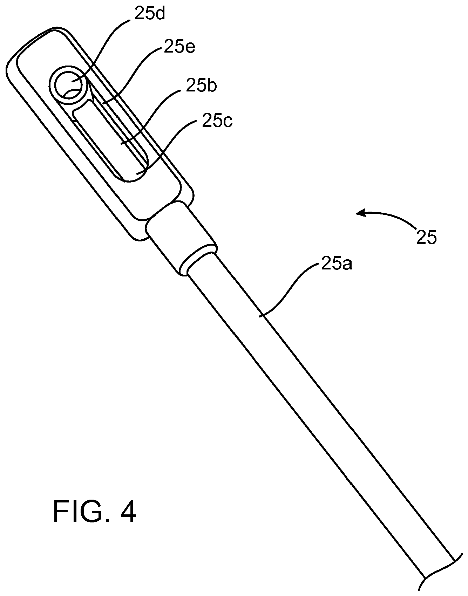

FIG. 4 provides a retractor cable 25 that is attached at its proximal end to the automated control unit. The retractor cable 25 contains an outer cable 25a and a wire 25c nested within the outer cable 25a. The distal end of the wire 25c contains a connection element 25b with a fitting 25d that is removably attachable to a post (not shown) protruding from the underside of the connection interface 24b. The connection element 25b is exposed through a window 25e at the distal end of the outer cable 25a. The proximal end of the wire 25c is connected to a device, such as a motor, within or attached to the automated control unit 40. The device can pull and push the wire 25c a distance that allows the connection element 25b to translate within the window 25e at least about half an inch to about three inches in length. The window 25e is at least about half an inch to about three inches in length. In one embodiment, the window 25e is about half an inch in length, and the connection element 25b translates about half an inch in length within the window as a consequence of the device moving the wire 25c a distance of about half an inch in length. Alternatively, the proximal end of the wire 25c is attached to a device, such as a motor, that coils the wire around a core such that the connection element 25b translates within the window 25e as the device coils and uncoils the wire around the core. When the fitting 25d is connected to the post of the connection element 24b, the connection element 24b translates proximally as the wire 25c is retracted by the device at the automated control unit 40. The wire 25c thus pulls the connection element 24b proximally, which retracts the retraction member 21, pulling the eyelid open. When the tension on the wire 25c is released by the device, then the pulling force on the connection element 24b is released and the connection element 24 translates in the distal direction allowing the retraction member 21 to extend and return to its resting position and allowing the eyelid to again close.

However, in other instances, it is to be understood that the force for moving the retraction portion 20 may be communicated via other suitable mechanisms, such as via a wireless automated control unit. In such an instance, the cable 25 and the attendant interfaces would not be necessary for retracting the retraction portion 20. In other instances, the automated control unit 40 may function to control the retraction of the retraction portion 20 through a cable 25 that transmits electrical signals.

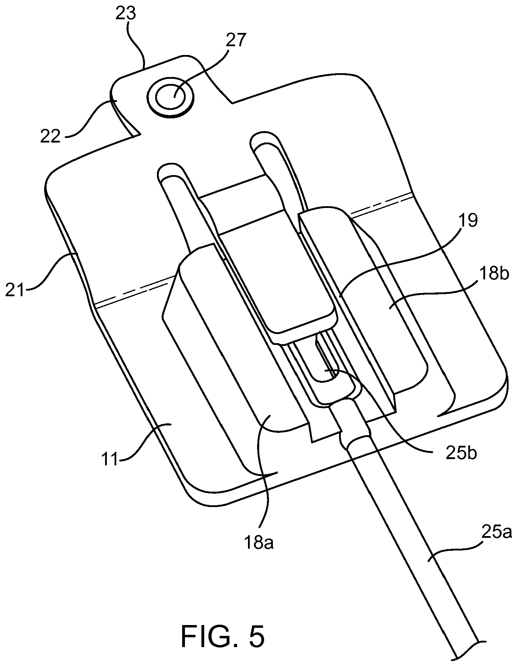

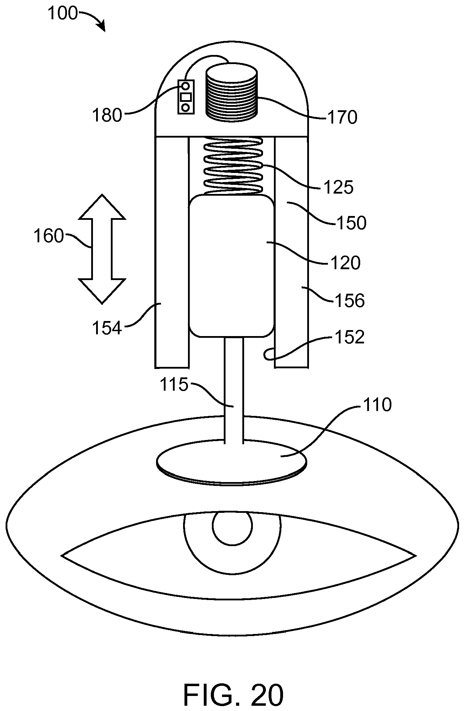

FIG. 5 provides a three-dimensional view of the retractor cable 25 of FIG. 4 when coupled to the eyelid retractor 1 of FIG 1. As can be seen with respect to FIG. 5, the eyelid retractor 1 includes an anchoring portion 10 having a base member 11, and a retraction portion 20 having a retraction member 21, and an attachment member 22. The attachment member 22 includes a calibration marker 27 on its top side. Alternatively, the attachment member does not have a calibration marker, and a separate calibration marker can be placed on the subject's eyelid. The retraction portion 20 further includes a channel interface member 24a having a connection interface 24b. The channel interface member 24a translates in a proximal and distal direction within a channel 17 of the anchoring portion 10. The channel 17 includes boundary members 18 a,b which form the channel 17 within which the channel interface member 24a translates. The connection interface 24b of the channel interface member 24a connects with a connection element 25b, through which connection the retraction of the retraction portion 20 may be controlled, for instance, by a suitable automated control unit 40. Other means for controlling the opening and closing of eyelids is describe further below with respect to Figures 20-22.

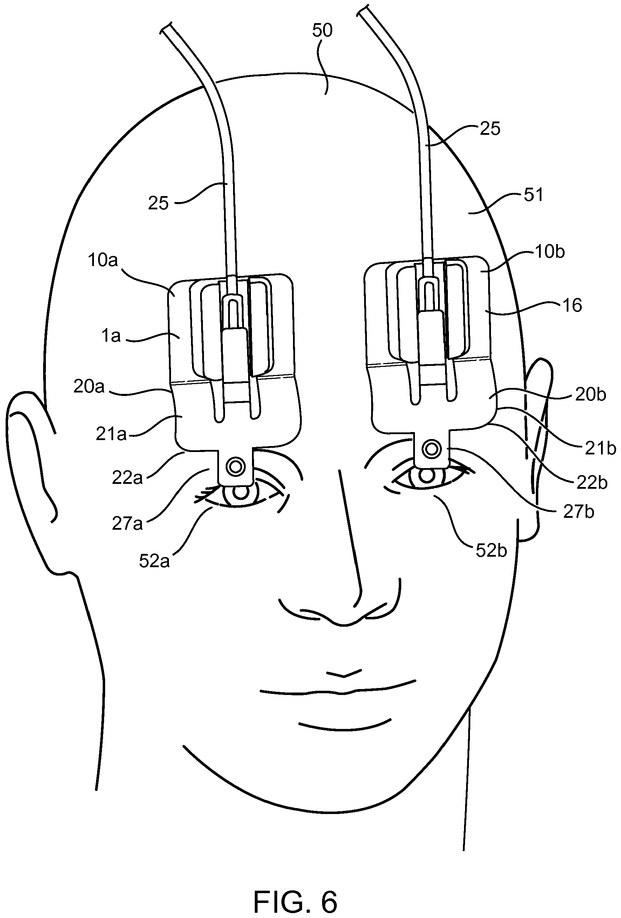

FIG. 6 is an illustration of a pair of eyelid retractors 1 a,b as they would be employed in adhering to the forehead 51 of a subject 50 over each of the subject's eyes 53 a,b. As can be seen with respect to FIG. 6 each eyelid retractor 1 a,b includes an anchoring portion 10 a,b and a retracting portion 20 a,b. The retraction portions 20 a,b each include retraction members 21 a,b having attachment members 22 a,b, which attachment members 22 a,b include calibration markers 27 a,b on their top sides. The eyelid retractors 1 a,b further include cables 25 for controlling the opening and closing of the eyelids 52 a,b, such as through interaction with a suitable controller 40. Using dual retractors as shown in Fig. 6 allows for binocular monitoring and analysis of the subject's eyes. Alternatively, calibration markers can be separate items that are independently and separately placed on a subject's eyelids.

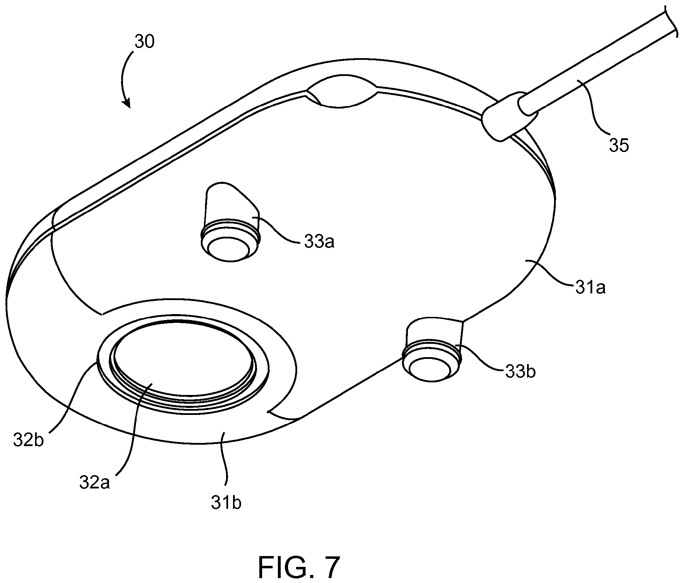

As indicated above, in certain instances, an eyelid retractor 1 is configured for being employed in conjunction with an ophthalmic instrument, such as a pupilometer 30, or a tonometer, a retinascope, a slit lamp bio microscope, an ophthalmoscope, or a keratometer. Accordingly, in one aspect, a pupilomter 30 is included within a system of the disclosure. FIG. 7 provides a three-dimensional view taken from the bottom of the pupilometer 30. As will be described in greater detail herein below, in certain instances, the pupilometer 30 may be configured such that a portion of the pupilometer 30 is capable of extending, e.g., axially, away from another portion of the pupilometer 30.

In such an instance, the pupilometer 30 may include at least a first housing 31 a, such as a housing 31a that includes one or more electronic components, and may further include a second housing 31b, such as a housing that includes an imaging sensor or camera 32a and/or a light source 32b. It is to be noted that although in this instance, the pupilometer 30 is configured as including two separable housings 31 a,b in certain instances, the pupilometer may only include a single housing, which housing contains both the electronic components, the imaging sensor(s), and the light source of the pupilometer 30. The pupilometer 30 additionally includes a communications cable 35 for communicating with another device, such as an automated control unit 40 of the disclosure.

Where the pupilometer 30 is to be employed as part of an automated pupil monitoring system, the pupilometer 30 may include support attachment members 33 a,b that are configured for allowing the pupilometer to be removably coupled to an eyelid retractor 1 of the disclosure, such as by interfacing with support members 16 a,b of an anchoring portion 10 of the eyelid retractor 1 of FIG. 1.

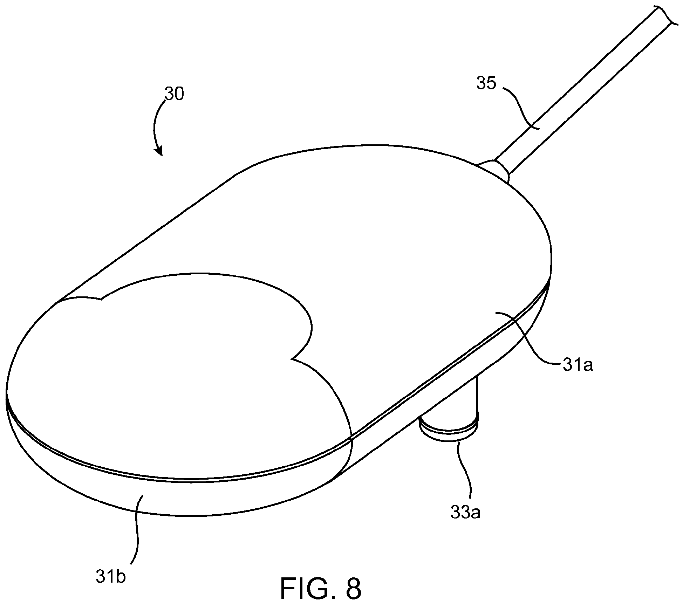

FIG. 8 provides a three-dimensional view of the top of the pupilometer 30 of FIG. 7. The pupilometer 30 includes a first housing 31a and a second housing 31b. The pupilometer 30 is in the closed, non-extended position. The pupilometer 30 additionally includes a support attachment member 33a, and a communications interface, e.g., cable 35, for communicating with an automated control unit 40, such as for controlling the functioning of the pupilometer and/or the eyelid retractor 1 and for storing and processing ocular data obtained by the pupilometer 30.

It is to be noted that although the pupilometer 30 is depicted as including a communications cable 35 for communicating with the automated control unit 40, in certain instances, this communication may be via wireless connection and rather than including a cable for transmission there between, the communications interface 35 will include componentry as is known in the art so as to enable wireless communication between the control unit 40 and the pupilometer 30 and/or eyelid retractor 1. In such a manner the control unit 40 may control the functioning of the pupilometer 30 and/or eyelid retractor 1 remotely, e.g., through wireless communication, such as by radio-frequency, Bluetooth, infra-red, or other such wireless communication technologies.

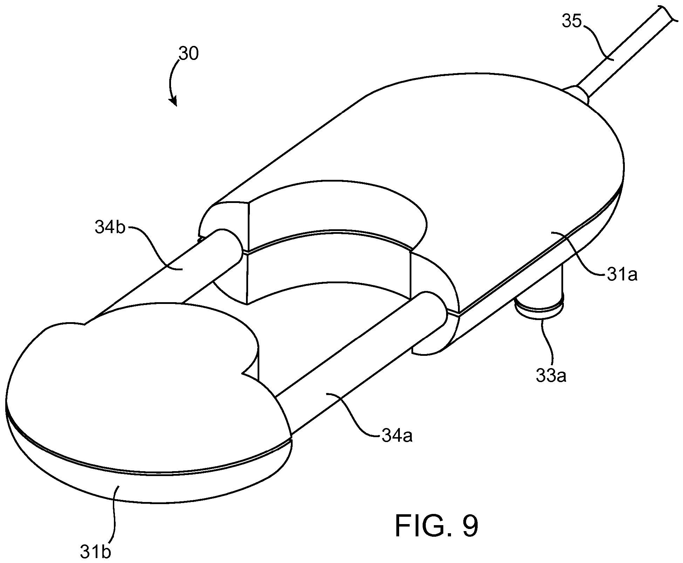

As set forth above and described with reference to FIG. 9, in certain instances, the pupilometer 30 may be configured such that a portion of the pupilometer 30 is capable of extending, e.g., axially, away from another portion of the pupilometer 30. For instance, in certain instances, it is useful to have a pupilometer 30 that can extend from a rest, closed position to an extended, open position so as to take measurements of the eye and then retract into the closed position after the measurements have been taken and/or the data processed.

In such an instance, as described herein, it might also be useful to have such an extendable/retractable pupilometer that can function in conjunction with a device, such as the eyelid retractor described herein, which eyelid retractor is capable of controlling the opening of the eyelid, for instance, when a measurement is to be taken by the pupilometer, and further closing the eyelid, once the appropriate measurement has been taken. A control unit40 may also be included, wherein the control unit signals the extending of the extendable portion of the pupilometer, the opening of the eyelid, e.g., the retraction of the eyelid retractor, the taking of the measurement, as well as the processing of the data, and the closing of the eyelid, e.g., the extending of the eyelid retractor, and the retraction of the pupilometer, e.g., once the measurement has been taken and/or the data has been obtained and/or processed.

Therefore, in certain instances, the pupilometer 30 is configured for moving from a closed, retracted position to an open, extended position, and is further configured for taking one or more measurements, and communicating those measurements to a control unit that may be associated with the pupilomter, e.g., via wire or wireless connection.

Accordingly, FIG. 9 provides a three-dimensional view taken from the top of the pupilometer assembly 30 of Figure 7 in an extended state. The pupilometer 30 includes a first housing 31 a, which housing includes the electronic components of the pupilometer, and further includes a second housing 31b, which housing includes the imaging sensor or camera 32a and/or a light source 32b.

In this embodiment, the pupilometer 30 further includes at least one extender, e.g., extenders 34a and 34b, depicted herein as rods, which extension rods 34a and 34b connect the first housing 31a with the second housing 31b and are configured for moving from a first, retracted position to a second, extended position, and thereby effectuating the linear movement of the second housing 31b away from the first housing 31 a, such as when it is desired to have the pupilometer take a measurement of a subject's eye. Accordingly, the extension rod(s) are configured for sliding in and out of the first housing 31a to permit the second housing 31b to be adjusted (manually or automatically) toward or away from the first housing.

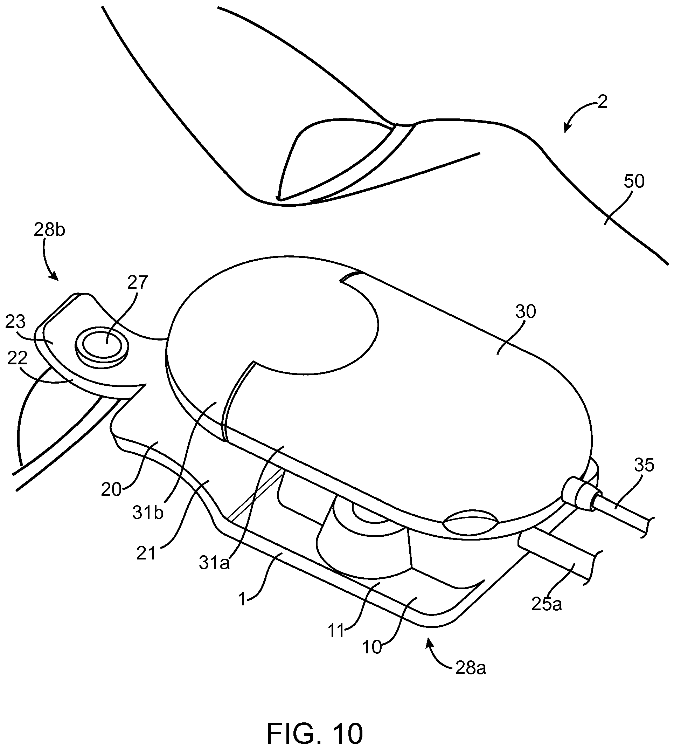

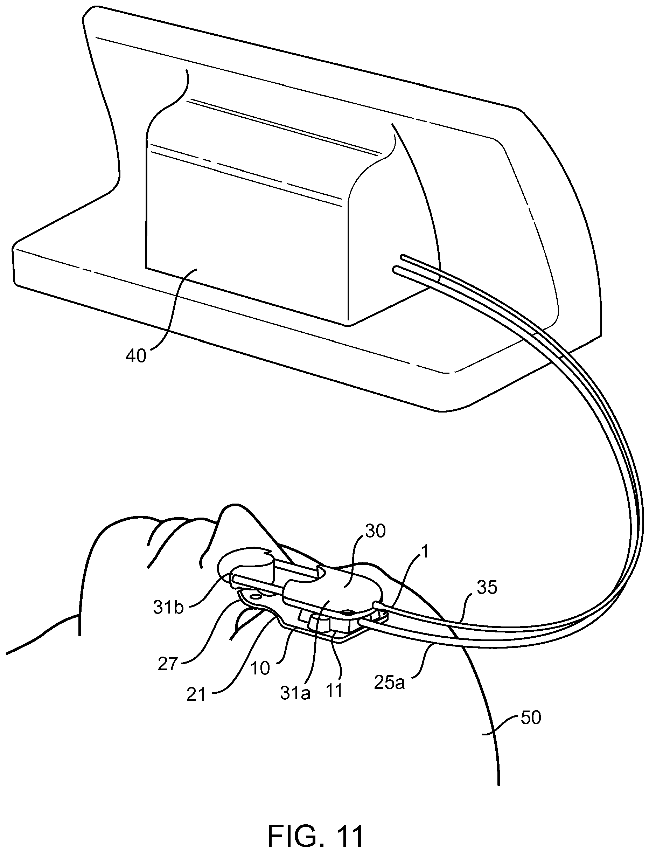

For instance, FIGS. 10 and 11 provide illustrations of the pupilometer 30 of FIG. 7 as it would be mounted onto the eyelid retractor 1 of FIG. 1 for use in a system of the disclosure, such as in conjunction with an automated control unit 40. As can be seen with respect to FIGS. 10 and 11, in one aspect, an automated pupil monitoring system 2 for monitoring the pupil of a subject 50 is shown. The monitoring system 2 includes an eyelid retractor 1, a pupilomter 30, and a control unit 40.

The eyelid retractor 1 includes an anchoring portion 10 and a retraction portion 20. The anchoring portion 10 includes a base member 11 which includes an attachment interface 28a. The attachment interface 28a includes an adhesive, suction cup(s) or other detachably anchoring component or material to detachably attach the base member 11 to the skin of the subject 50. The retraction portion 20 includes retraction member 21 having an attachment member 22, which attachment member 22 includes a calibration marker 27 on its top side. The attachment member 22 further includes an attachment interface 28b on its bottom side to attach the attachment member 22 to the eyelid 52 of a subject 50. The attachment interface 28b can have an adhesive material, suction cup(s) or other detachably anchoring component or material to detachably attach the attachment member 22 to the eyelid 52 of the subject 50.

As can be seen with respect to FIG. 10, the pupilometer 30 is coupled to the eyelid retractor 1. The support attachment members 33a,b of the pupilometer 30 are coupled to the support members 16a,b of the eyelid retractor. The pupilometer 30 includes a first portion or housing 31a and a second portion or housing 31b. The pupilometer is in the retracted, or closed position. As can be seen with respect to FIG. 11, the first portion 31a of the pupilometer 30 is configured for being coupled to an eyelid retractor 1, and the second portion 31b is configured for being extended away from the first portion 31a. The pupilometer includes rods 34 a,b for translating the second portion 31b away from the first portion, e.g., in a linear motion. The second portion 31b includes an imaging sensor or camera and a light source that emits light in the form of a flash so as to stimulate the pupil. The pupilomter 30 is in the extended, or open position.

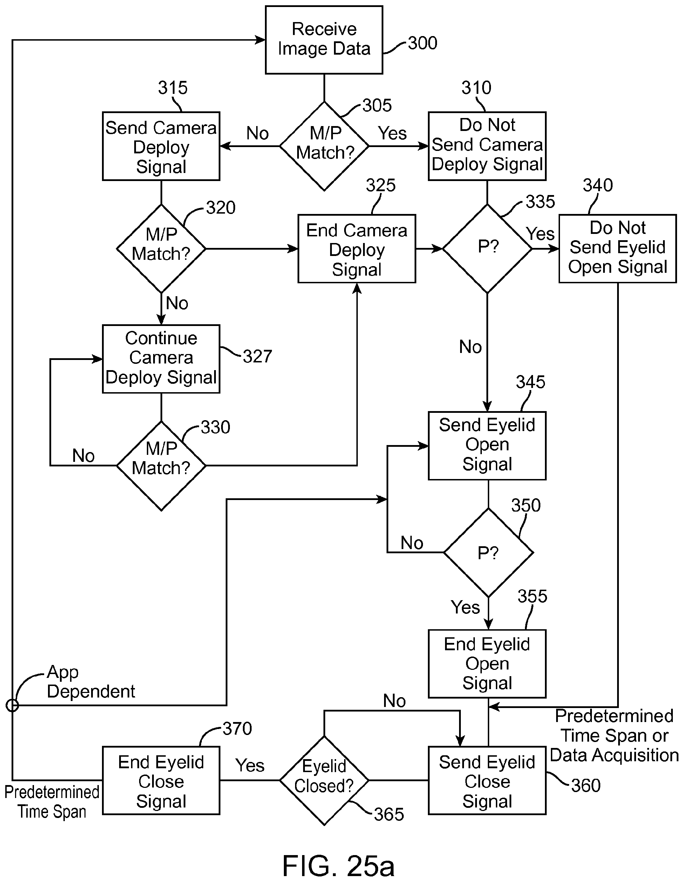

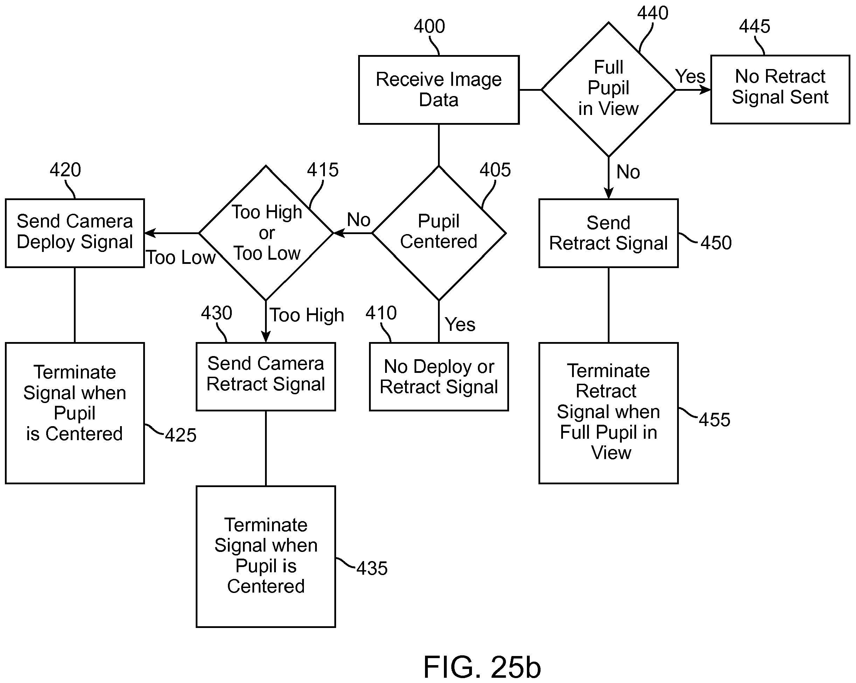

Accordingly, when the pupilometer 30 is in the extended position, as in FIG. 11, the second portion 31b is aligned with the pupil of a subject 50 and capable of flashing a light from its light source 32b so as to stimulate the pupil and to take both static and dynamic measurements of the pupil's response to the flash of light. The pupilometer is capable of receiving image data from the pupil, which data is communicated to an automated control unit 40. Hence, the system 2 may include an automated control unit 40, which control unit 40 may be in communication with the eyelid retractor 1, e.g. via connector cable 25, and the pupilometer 30, e.g., via communication cable 35. The movement of the pupilometer and the eyelid retractor can be controlled by software that automates the activity of these two units. The steps performed by such software are described in more detail below with respect to Figures 25a and 25b. The software can either be integrated into the pupilometer 30, the control unit 40, or another external source.

In certain embodiments, the automated control unit 40 controls the extending and retracting of the eyelid retractor 1, and further controls the extending and contracting of the two portions of the pupilometer 30, e.g., in conjunction with one another. For instance, in certain instances, the control unit 40 may include programing that allows the pupilometer to determine whether the eyelid is opened or closed, and whether or not to take a measurement of the pupil, i.e., when the eyelid is open.

Further, the programming may additionally control the closing of an open eyelid, such as after a measurement has been taken. Accordingly, the control unit 40 may include programing that controls the eyelid retractor 1 to open the eyelid, and/or to determine that the eyelid is open (e.g., by identifying the functioning of the eyelid retractor, sensing the pupil, or the like), and/or extend the pupilometer 30, and/or to take a measurement therewith, and/or to control the eyelid retractor 1 to close the eyelid, and/or to retract the pupilometer 30.

Such programing and control mechanisms may be important to protect the pupil and/or to ensure that the cornea does not dry out, which could cause damage to the pupil and/or cornea. An alarm may also be included so as to indicate that the eyelid should be opened and/or should be closed. It is to be noted that any of the steps recited herein can be performed automatically by the automated controller or manually by a user. Hence, the programming of the control unit 40 and/or pupilometer 30 may function to control the opening and closing of the eyelids, take and/or analyze measurements of the pupil, calibrate the system 2 and/or its components, and/or determine if the eyelids are open or closed and to warn a user of such.

In certain embodiments, such as that depicted in FIG. 11, the automated control unit 40 is configured so as to communicate with the pupilometer and/or eyelid retractor through one or more data and/or electric cables, e.g., 25 and 35. In other instances, this communication is configured so as to be through a wireless interface, e.g. wireless communication. Accordingly, the automated control unit may be configured for receiving and analyzing data received from the pupilometer, so as to provide an output of the analyzed data, such as data pertaining to a relative afferent papillary defect, optical nerve damage, intra-ocular pressure, a neurological condition, and the like. Hence, the automated control unit 40 may include software that receives data from one or more pupilometers 30, analyzes that data, and provides an output pertaining to the analyzed data, such as compiled data indicative of a relative afferent papillary defect, optic nerve disease, intra-ocular pressure, a neurological condition, and the like.

As set forth above, the eyelid retractor 1 may include a size calibration marker 27, wherein the size, e.g., absolute size (diameter, circumference and/or any other value representative of the marker's absolute size), of the calibration marker 27 may be contained in a memory of the pupilometer 30, and/or in a memory of the control unit 40. The distance from the camera 32a to the marker is not known. The camera detects the marker and the microprocessor recalibrates the pupilometer based on the detection of the marker and relative size of the marker compared to its actual known size. The data representing the absolute size of the calibration marker 27 can be accessed by any program that is run by a central processing unit contained in the pupilometer 30 or the control unit 40. The thickness of the attachment member 22 can also be accessibly programmed into the memory of the pupilometer or a memory of the control unit 40, such that data representing the size of thickness of the attachment member 22 can be accessed by any program that is run by a central processing unit contained in the pupilometer 30 or the control unit 40. Accordingly, the pupilometer 30 and/or the control unit 40 may include pupil size determination software that processes data representative of the absolute size of the size calibration marker 27, and the relative size of the marker, obtained from the imaging sensor or camera 32a of the pupilometer 30, to recalibrate the pupilometer 30 and then calculate the actual or absolute size of the subject's pupil. The pupilometer 30 and/or control unit 40 is therefore capable of determining an actual or absolute size of the pupil of a subject. In one embodiment, the camera 32a obtains an image of the calibration marker 27 and determines a relative size of the calibration marker 27. The relative size of the marker 27 is compared to its actual known size using calibration software in the pupilometer 30 or control unit 40, and a distance between the camera 32a and the marker 27 is calculated by the software. The eyelid retractor 1 then retracts the eyelid of the patient and the camera 32a obtains an image of the subject's pupil with a relative size of the pupil. Using the calculated distance between the camera 32a and the pupil, the calibration software then calculates an absolute size of the subjects pupil based on the relative size and the calculated distance and calibrates the pupilometer accordingly. In another embodiment of a calibration method and software, calibration software compares the actual size of the calibration marker 27 and its relative size and calculates a calibration ratio. The calibration ratio is applied to the relative size of the subject's pupil to calculate an absolute size of the pupil and to calibrate the pupilometer accordingly with respect to measurements obtained after the calibration.

Additionally, the pupilometer 30 and/or control unit 40 may further include software capable of determining when the eyelid(s) of a subject is opened or closed; controlling the opening and closing the eyelids by activating and deactivating the eyelid retractor 1; controlling the deployment and retraction of the pupilometer 30. The software is dependent on data it receives from the pupilometer 30. The software can be included in a hard drive within the pupilometer 30 or the control unit 40, or be part of any computer program product embodied in a non-transitory computer-readable storage medium and having computer-executable instructions recorded on said storage medium for controlling the eyelid retractor 1 or 200 and the pupilometer 30. Examples of non-transitory computer-readable storage mediums, include, but are not limited to, external or internal computer hard drives, thumb drives, CD-ROMs, DVDs, floppy disks, ROM memory, RAM memory, and the like.

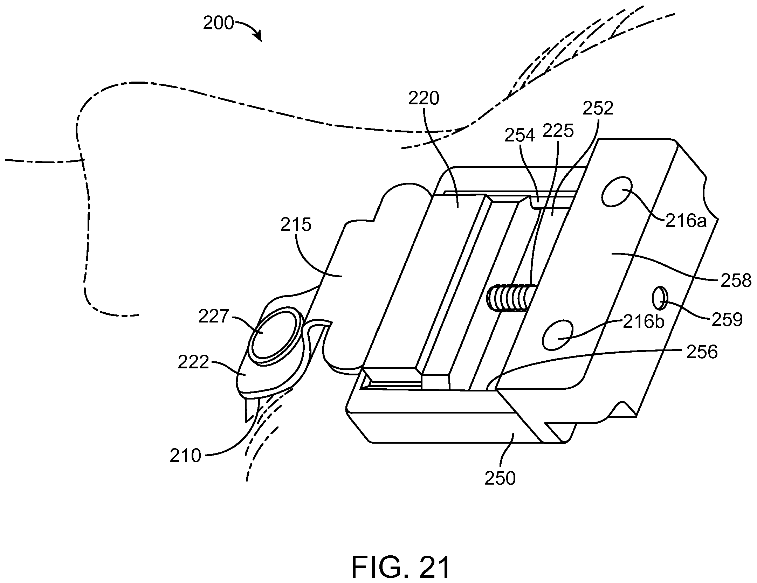

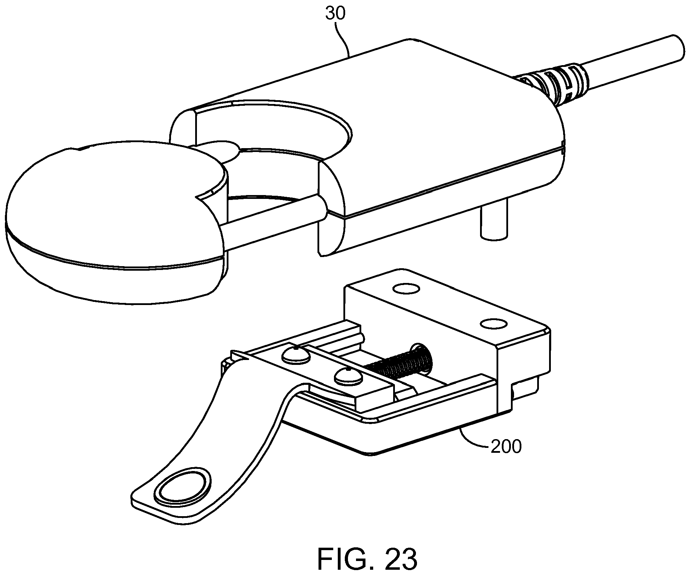

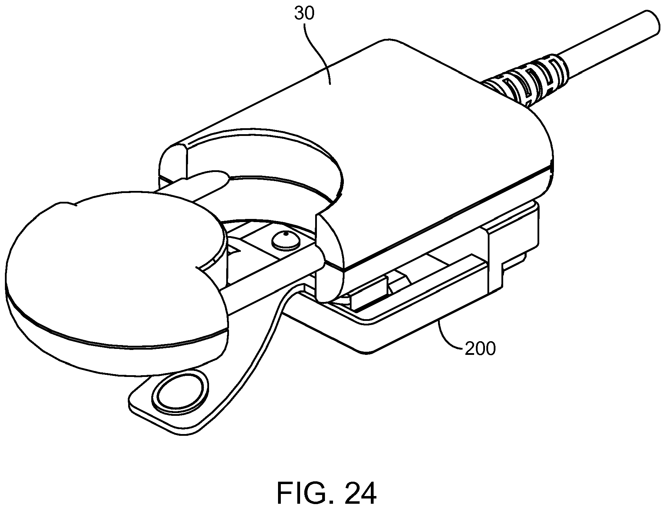

The steps performed by such software are described in more detail with reference to Figures 25a and 25b. The software is executable by a CPU within the control unit 40 or the pupilometer 30. The software enables a completely automated system in which the retractor 1 (or retractor 200 shown in Figures 21-24) and pupilometer 30 (or other ophthalmic instruments containing a camera) work together in an automated fashion to monitor the eyes of a subject while the subject is in a coma, undergoing a surgical procedure and is therefore unconscious, or is otherwise incapacitated. The automated method is performed as follows and is described with reference to retractor 1 of Figures 1-5, but is applicable equally to the retractor 200 in Figures 21-24). The eyelid retractor 1 attachment interface 28a is secured to the subject's forehead, and the attachment interface 28b of the attachment member 22 is secured to the subject's eyelid 52. Alternatively, the eyelid retractor has an attachment portion that is adapted for attaching to a bed post or other stationary object, such as a stationary control unit. The pupilometer 30 is then mounted to the top of the eyelid retractor 1 as shown in Figures 10 and 24. The CPU, software, and camera are activated and the means for controlling the movement of the retraction portion 20 (or 220 in Figures. 21-24) relative to the anchoring portion 10 (or 210 in Figures 21-24) is also activated or at least powered. The CPU begins to receive image data from the camera 32a of the pupilometer, such as digital image data. The pupilometer 30 is in its retracted position initially, and the camera 32a begins to send image data to the CPU. As shown in Figure 25a, the CPU receives the image data at step 300. The software matches the data against preprogrammed image data associated with the calibration marker 27 or a pupil (or other identifying indicia) of an eye and determines whether the images are of the marker 27 or a pupil (or other identifying indicia) at step 305. The preprogrammed image data can be data preprogrammed by the factor, or it can be image data obtained by the camera of the actual patient or subject before step 300. The camera can be used before step 300 to obtain an image of the marker 27, or the subject's eye, pupil, eyelilds, eyelashes, cornea, sclera, or other identifying indicia of the subject's eye, and that data can be transmitted to the control unit and saved in memory and used as the preprogrammed comparator image data. If the received image data match the preprogrammed marker 27 or pupil data, then at step 310 no signal is sent to the pupilometer to extend the rods 34a and 34b outward. If the image data do not match the preprogrammed pupil or marker 27 data, a "camera deploy" command and associated signal is sent at step 315 to the pupilometer 30 to cause the extenders 34a and 34b to begin extending out from the first housing 31a. The first housing 31a contains electronics for receiving signals from the CPU and powering the movement of the extenders 34a and 34b. The camera 32a continues to send image data and the CPU continues to receive that data as the extenders 34a and 34b extend out of the housing 31a. The camera 32a continues to obtain images and send image data to the CPU. The software continues to analyze the image data received from the camera at step 320 and if there is a match with the preprogrammed marker 27 or pupil data, then an "end camera deploy" command and associated signal is sent to the pupilometer 30 at step 325 to stop extending the extenders 34a and 34b. If there is no match with the preprogrammed marker 27 or pupil data, the camera deploy signal continues at step 327, and this process is continuously looped until there is a match with the preprogrammed marker 27 or pupil data (or eyeball data in yet another embodiment)a at step 330, and then the "end camera deploy" command and associated signal is sent to the pupilometer 30 at step 325. Thus, once the camera 32a has found the marker 27 or the subject's pupil (or eyeball) (i.e., the marker 27 or substantially all of the pupil (or eyeball in another embodiment) is within the field of view of the camera), the extenders 34a and 34b stop extending and the camera stops at that location. The camera 32a continues sending image data to the CPU and the software now compares thatdata to preprogrammed data associated with a pupil (or eyeball) at step 335. If the received image data match preprogrammed pupil (or eyeball) image data (i.e., the substantially all of the pupil (or eyeball in another embodiment) is within the field of view of the camera) then at step 340 the software does not send a command to activate the eyelid retractor (1 or 200) to open the eyelid of the subject (or alternatively, it sends a negative command to not retract the eyelid retractor). If the data do not match , i.e, there is no pupil (or eyeball) within the field of view of the camera or substantially all of the pupil (or eyeball) is not within the field of view of the camera, then an "open eyelid" or "retract" command and associated signal is sent to the eyelid retractor 1 or 200 at step 345. The camera 32a continues sending image data to the CPU and the software continues comparing thatdata to preprogrammed data associated with a pupil(or eyeba110 at step 350. If the received data do not match the preprogrammed data, i.e., there is no pupil (or eyeball) within the field of view of the camera 32a or substantially all of the pupil (or eyeball) is not within the field of view of the camera, then the "open eyelid" signal continues in a looped fashion as shown in Figure 25a. If the image data matches the preprogrammed pupil data (i.e.,substantially all of the pupil (or eyeball) is within the field of view of the camera 32a) then at step 355 the "open eyelid" command is terminated, and the signal to retract the eyelid retractor 1 or 200 ceases. In one embodiment, the "open eyelid signal" at step 345 can be overridden and the signal terminated if a certain pressure gradient is reached, and this can be determined by incorporating a pressure sensor into the eyelid retractor. In this case, the pressure sensor is coupled to the eyelid retractor such that the pulling force of the eyelid is communicated to the pressure sensor on the eyelid retractor. The eyelid pulling force data is communicated to the CPU and when a threshold pulling force is reached, any signal to retract the eyelid at step 345 will be overridden and the "open eyelid" signal will be terminated. The eyelid retractor keeps the eyelid open for a predetermined and set amount of time, such as for one, two, three, four, five, six, seven, eight, nine, ten or more minutes. Alternatively, the eyelid retractor keeps the eyelid open until the pupilometer obtains a set of data, such as pupil reflex data, such that the retractor remains retracted until the data is obtained irrespective of the amount of time it takes to obtain the data. For example, the retractor can keep the eyelid open until the pupilometer sends a flash of light to the pupil, images of the pupil's response are transmitted to a control unit and processed, and the processor determines that the image data have been properly processed. During this period, the camera can be continuously obtaining image data from the subject's eye, which is saved in a memory of the pupilometer 30 or the control unit 40, or an external computer or data storage device in communication with the pupilometer 30 or control unit 40. Once the predetermined amount of time expires (or the data is properly obtained), an "eyelid close" command and associated signal is sent at step 360 to the eyelid retract 1 or 200 and the retractor is allowed to return gradually to its extended position allowing the eyelid to gradually close. During this process, image data are sent to the CPU and the software compares thatdata against preprogrammed image data of an eyelid or other ocular marker, such as eyelid and eyelashes, or just eyelashes and once there is a match an "end eyelid close" command and associated signal is sent at step 370 to the eyelid retractor and the eyelid retractor stops extending. Until there is a match between the received image data and the preprogrammed image data , the send eyelid close signal will continue in a looped fashion as shown in Figure 25a. The eyelid remains closed for a predetermined amount of time, such as for one, two, three, four, five, six, seven, eight, nine, ten or more minutes. Then process returns to either step 345 or to step 300, depending on the application of the system and the process starts over again either from step 300 or step 345.

As shown in Figure 25b, the software is able to process the image data received from the camera to make fine adjustments to the position of the camera and the retraction/extension of the eyelid retractor, so that if the eyelid retractor has slipped and the eyelid has closed prematurely, an "eyelid open" command and associated signal will be sent to the retractor until the pupil of the eye is again in view of the camera, such that all or substantially all of the pupil is within the view of the camera. Also, if the pupilometer needs to be extended or retracted even a slight amount as a result in the change in shape or size of the pupil or movement of the subject's head relative to the camera, the software is able to send a camera deploy or camera retract signal to move the camera forward or backward so that the pupil is again within the view of the camera, such that all or substantially all of the pupil is within the view of the camera. In embodiment, commands and associated signals are sent to the camera and retractor to keep the pupil substantially within the center of the field of view of the camera, such that if the pupil is not substantially within the center of the field of view of the camera, either the camera location will move or the retractor will readjust. To these ends, the CPU receives image data at step 400. If the pupil is substantially within the center of the field of view of the camera at step 405, then no deploy or retract signal is sent to the pupilometer at step 410. If the pupil is not substantially within the center of the field of view of the camera then the software analyzes the image data at step 415 and determines whether the pupil is too high or too low within the field of view of the camera. If the pupil is too low within the field of view of the camera, then a "deploy camera" command and associated signal is sent to the pupilometer at step 420 to extend the camera forward or in the inferior direction along a human subject's body. Once the the pupil is substantially within the center of the field of view of the camera, the "deploy camera" signal is terminated at step 425. If the pupil is too high within the field of view of the camera, then a "retract camera" command and associated signal is sent to the pupilometer at step 430 to retract the camera backwards or in the superior direction along a human subject's body. Once the pupil is substantially within the center of the field of view of the camera, the "retract camera" signal is terminated at step 435. At step 440, the software analyzes the image data to determine if afull pupil is in the field of view of the camera, and if it is then at step 445 no retract signal is sent to the eyelid retractor. If the software determines that a full pupil is not within the field of view of the camera, then a retract signal is sent to the eyelid retractor at step 450. Once a full pupil is within the field of view of the camera, the retract signal is terminated at step 455. In addition, the retract signal may also be terminated if a certain pressure gradient is reached, and this can be determined by incorporating a pressure sensor into the eyelid retractor. In this case, the pressure sensor is coupled to the eyelid retractor such that the pulling force of the eyelid is communicated to the pressure sensor on the eyelid retractor. The eyelid pulling force data is communicated to the CPU and when a threshold pulling force is reached, any signal to retract the eyelid will be overridden and the "retract" signal will be terminated.

The computer architecture of the software in relation to the control unit and the pupilometer 30 and eyelid retractor 1 (or 200) is as follows. The camera 32a on the pupilometer 30 is directly connected to a CPU interface, and the CPU is either within the pupilometer itself, or within an external control unit 40. The image data from the camera 32a is input into the CPU frame by frame (one frame at a time). For example, VGA resolution that is 640 x 480 pixels for the images can be used. The CPU contains the software that analyzes the data and issues the commands in accordance with command steps described in Figures 25a and 25b. Based on the image data analyzed by the software, the CPU makes a decision on what command and associated signal to issue and send the appropriate signal to an I/O (input/output) of the CPU. That I/O is connected to the electronics that controls the motors (Figures 1-5) of the eyelid retractor or the magnetic coil (Figures 20-23) of the eyelid retractor 200, and the motors/actuators that control the extenders 34a and 34b of the pupilometer 30. For example, when the pupilometer 30 is looking for a marker 27, in a memory of the CPU (or in a memory communicating with the CPU) is a preprogrammed description of how the marker 27 looks and the software will match the incoming image data that is received from the camera 32a to that preprogrammed data. Another example is that when the pupilometer 30 is looking for the pupil the characteristics of the pupil, such as reflections of the LED light(s) (from pupiometer) from the cornea or the border of the pupil/iris are known. Based on those features, the software will decide if it has detected the pupil and will make the appropriate decision of what signals to send to the I/O.

The above software and associated method can be applied to a binocular system with two eyelid retractors, such as shown in Figure 6 with a pupilometer mounted to each eyelid retractor, and the software controlling each eyelid retractor and its pupilometer independently or in a coordinated fashion. For example, in one embodiment, a computer program coordinates the two retractors and pupilometers so that the pupilometers are positioned over opened eyes and imaging both pupils at the same time with stimulating lights being flashed to each eye simultaneously, or where a flash is issued to one eye but image data is obtained and recorded by both pupilometers at the same time, thus allowing the pupilometers to gather data relating to anisocoria or also relating to relative afferent pupillary defect.

The above software and associated methods can also be used with an ophthalmic instrument other than a pupilometer (such as those described herein). In that case, the ophthalmic instrument will still contain a camera, but instead of the pupil as the biomarker of choice, some other ocular indicia can be used or the pupil can also still be used.

In certain embodiments, such as that described with reference to FIG. 6, an automated pupil monitoring system of the disclosure may include a first and a second eyelid retractor which are configured for being adhered to skin of the subject adjacent to respective eyes of a subject. In such an instance, a first and a second pupilometer may be provided, wherein the pupilometers are coupled to the first and second eyelid retractors. Each of the first and second pupilometers, therefore, may include an imaging sensor or camera and a light emitter so as to emit light, e.g., in the form of a flash, so as to stimulate the pupil and to obtain data thereby. The automated control unit would therefore be in communication with both the first and second eyelid retractor and/or the first and second pupilometers so as to compile and analyze, e.g., compare, the data obtained from both eyes. The automated control unit 40 may also have programming allowing it to control the first and second pupilometers 30 and the first and second connection elements 25b that are connected to the eyelid retractors. In certain embodiments, such a system may include two or more control units 40 that may be configured for communicating with one another. The control unit(s) 40 can be a personal computer or other device with a central processing unit containing software designed to control the connection element(s) 25b and the pupilometer(s) 30, and further containing a memory to store ocular data obtained by the pupilometer 30.

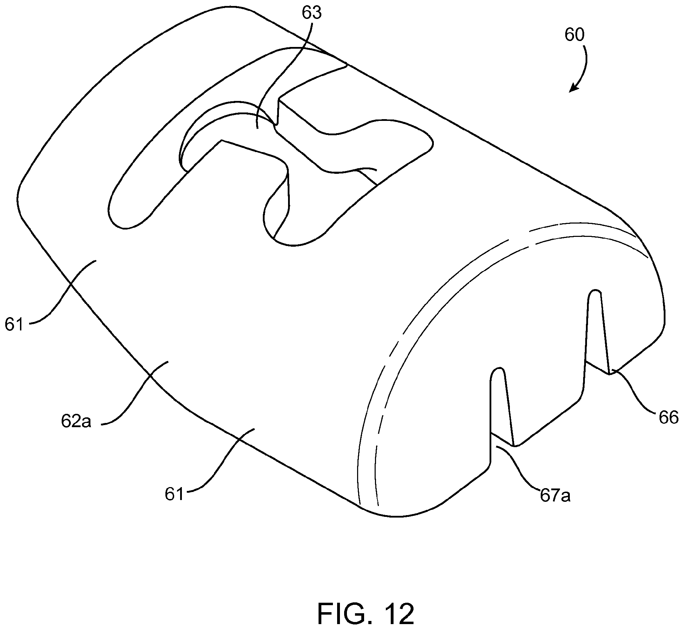

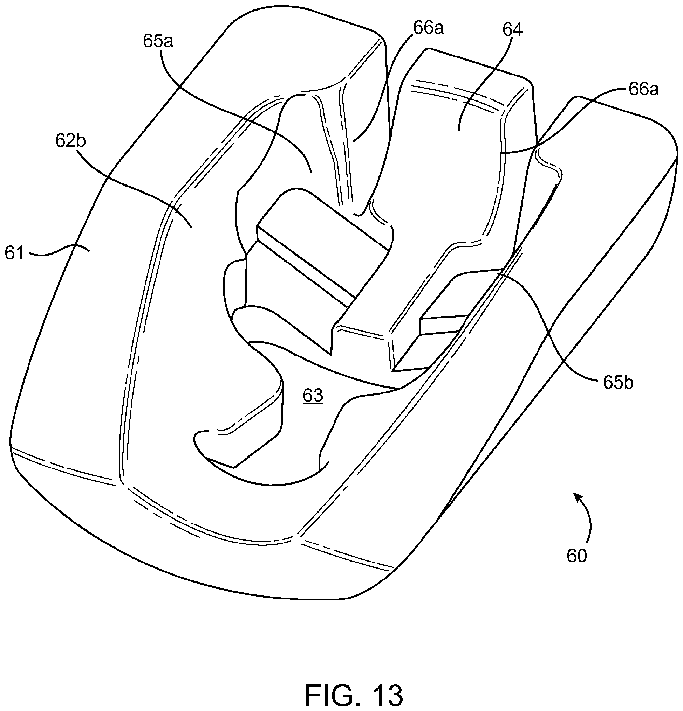

In accordance with another aspect of the disclosure, and as can be seen with respect to FIGS. 12 and 13, a three-dimensional view of a surgical face mask 60 for use by a subject during a surgical procedure is provided. The surgical face mask has a casing 61 with an interior surface 62a and an exterior surface 62b. The casing 61 has at least one aperture 63 that communicates through the casing 61. The aperture 63 can be positioned in the casing 61 to allow visualization of the subject's eyes and forming an access therethrough to the subject's nose and mouth. A forehead rest 64 in the interior surface 62a of the casing 61 divides the interior of the casing 61 into a left ocular region 65a and a right ocular region 65b, each ocular region 65 a,b forming an indentation 66 a,b respectively that is sized to accommodate an eyelid retractor 1 placed on a forehead and eyelid of the subject 50. The mask 60 further includes cable access channels 67 a,b at the top of the casing 61, which access channels allow respective communication cables to pass there through

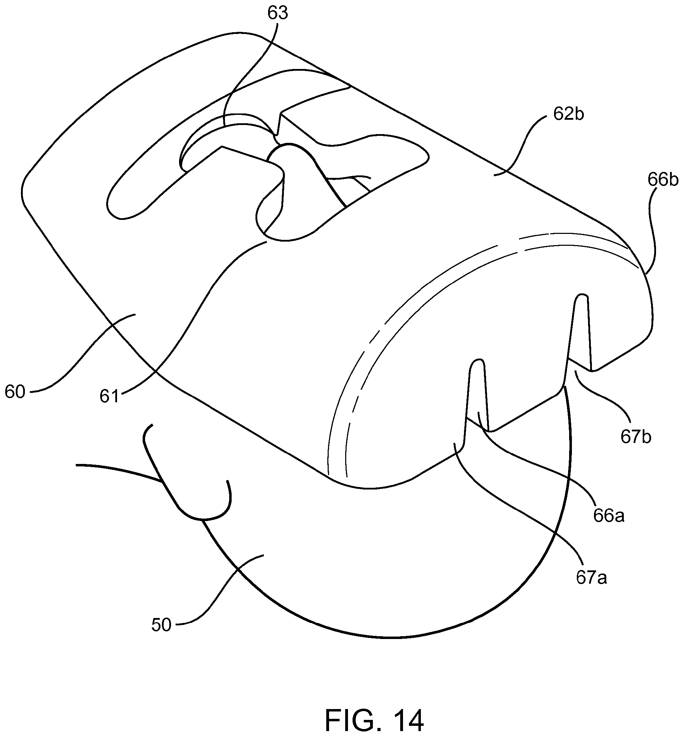

FIG. 14 provides an illustration of the surgical face mask 60 of FIG. 12 as worn by subject 50. The interior surface 62a of the surgical face mask 60 rests against the face of a user 50. The exterior surface 62b is facing the outer side of the face mask 60. The face mask 60 includes an aperture 63 that communicates through the casing 61 and further includes a left ocular region indentation 66a and a right ocular region indentation 66b.

Indentations 66 a,b encase respective eyelid retractors 1 a,b that have been placed on a forehead and eyelid of the subject 50, and pupilometers 30 a,b that have been coupled to the eyelid retractors 1 a,b. Their respective communication cables can be seen as they pass through cable access channels 67 a,b at the top of the casing 61. In certain embodiments, the surgical mask 60 may further include a strap that may be connected to one side of the casing and a fastener that may be connected to the other side of the casing that receives the strap so as to secure the casing 61 in place. The strap goes around the subject's head to secure the mask 60 to the subject's face. Other fastening mechanisms may also be employed.

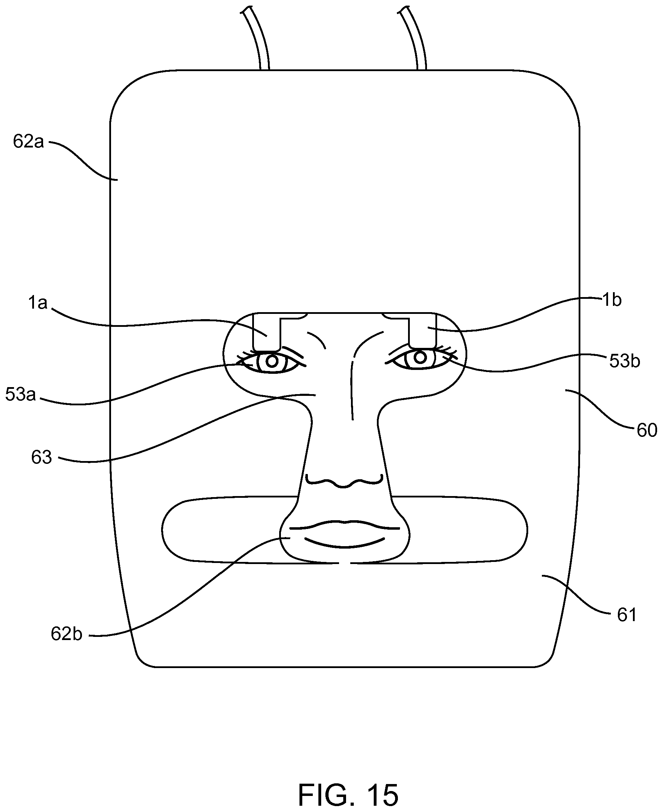

FIG 15 provides a top view of the surgical face mask 60 of FIG. 12 as worn by a subject 50 during a surgical procedure in which the subject is on his or her back. The casing 61 has an interior surface 62b and an exterior surface 62a. The face mask 60 includes an aperture 63 that communicates through and is positioned in the casing so as to allow visualization of the subject's eyes 53 a,b. The aperture 63 further forms an access to the subject's 50 nose and mouth. The surgical face mask 60 includes two ocular regions 65 a,b which form indentations 66 a,b that are sized to accommodate eyelid retractors 1 a,b that have been placed on the forehead and eyelid of the subject 50. The distal portions of the eyelid retractors 1 a,b can be seen through aperture 63.

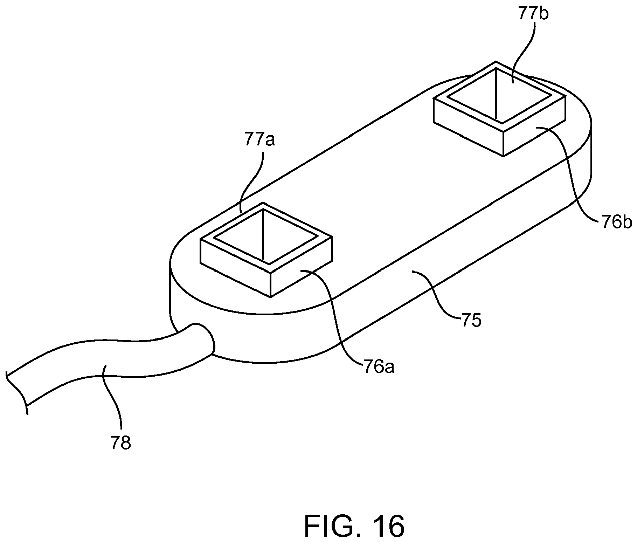

FIG. 16 provides a three-dimensional view of a pupilometer with an imaging sensor or camera 75 having binocular imaging sensors or cameras 76 a,b positioned thereon. The pupil imaging sensors 76 a,b are spaced apart and configured for being aligned with the pupils of the eyes of a subject and for taking measurements thereof. The distance between the pair of binocular imaging sensors may be adjustable. The binocular imaging sensors 76 a,b may further include a pair of binocular light sources 77 a,b that can emit light in the form of a flash to stimulate the pupil. The sensor 75 additionally includes a communications cable 78 that is configured for communicating with a control unit 40 of the disclosure. The control unit 40 can be a personal computer or other device with a central processing unit containing software designed to control the connection element 25b and the pupilometer 30, and further containing a memory to store ocular data obtained by the pupilometer 30.

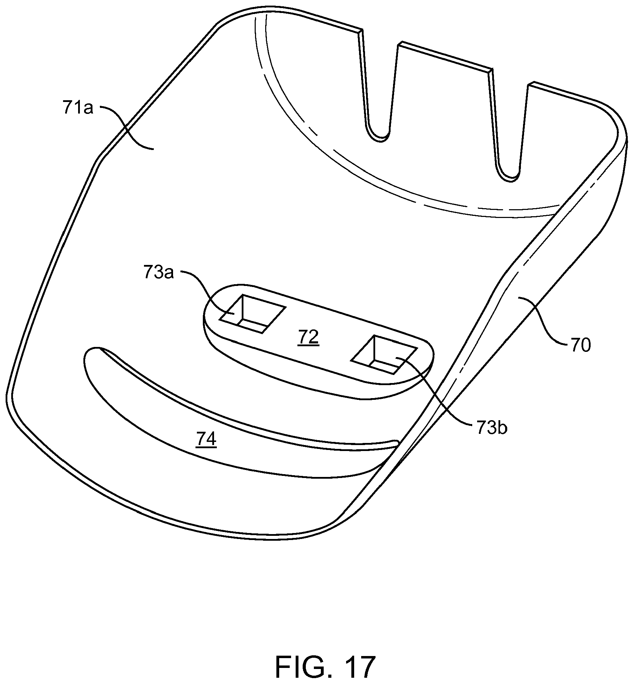

As can be seen with respect to FIG. 17, in certain embodiments, a retention casing 70 may be provided. FIG. 17 provides a three-dimensional view taken of the interior surface 71a of an optional surgical face mask retention casing 70. The retention casing 70 is configured for being positioned over a face mask 60 of the disclosure so as to secure the position of the face mask 60 relative to the subject's head 50. The retention casing 70 further includes a sensor receiving receptacle 72 that is configured for receiving the binocular imaging sensor 75 of FIG. 16. The receiving receptacle 72 of the retention casing 70 includes apertures 73 a,b that pass through the casing and align with the pupil imaging sensors 76 a,b when the imaging sensor 75 is appropriately received within the receptacle 72. Additionally, when properly aligned within the casing 70, the binocular imaging sensors 76 a,b of the sensor 75 are aligned with the pupils of the eyes of a subject and configured for taking measurements thereof.

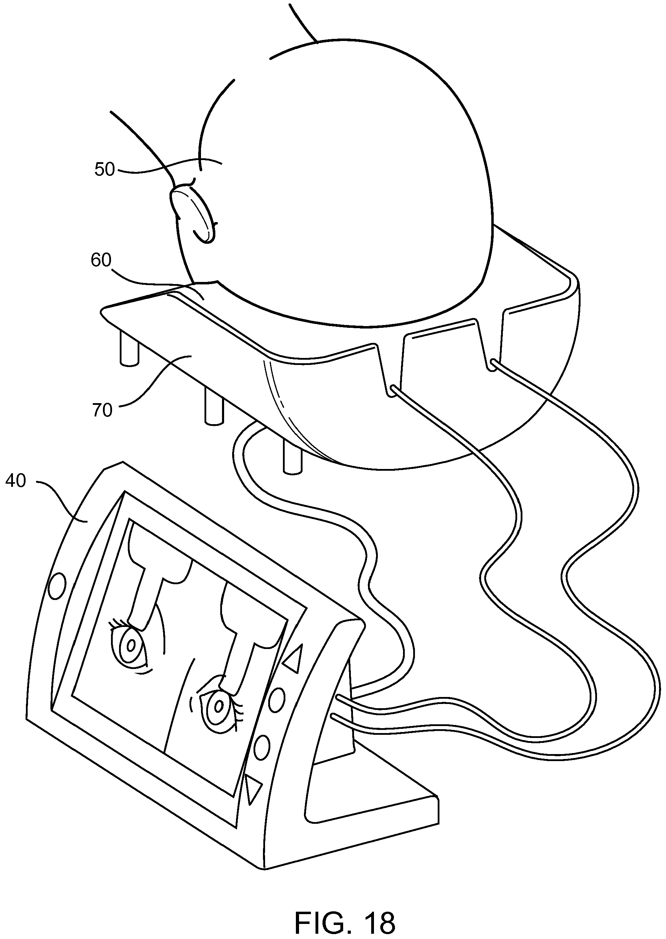

FIG. 18 provides an illustration of an ocular monitoring system that includes the surgical face mask 60 of FIG. 12, the pupilometer with imaging sensor 75 of FIG. 16, and the surgical face mask retention casing 70 of FIG. 17. The ocular monitoring system includes a plurality of eyelid retractors 1 a,b that are each associated with respective pupilometers 30 a,b all of which are in communication with an automated ocular monitoring control unit 40 during a surgical procedure.

Accordingly, in accordance with another aspect of the disclosure, an automated pupil monitoring system for monitoring the pupil of a subject is provided. The automated pupil monitoring system may include one or more of the following: eyelid retractors 1 a,b; an automated control unit 40; a surgical face mask 60; and, in this embodiment, a pupilometer with imaging sensor or camera 75 in conjunction with a surgical face mask retention casing 70.

As depicted, the head 51 of a subject 50 is positioned within the surgical face mask 60, eyelid retractors 1 a,b with associated pupilometers 30 a,b, are attached to the subject 50, and appropriately positioned within the face mask 60. The pupilometer imaging sensor 75 is positioned within the receptacle 72 of the retention mask 70, which retention mask 70 is coupled to the face mask 60 in such a manner that the binocular imaging sensors 76 a,b of the sensor 75 are aligned with the pupils of the eyes 53 of the subject.

The eyelid retractors 1 a,b; pupilometers 30 a,b; and imaging sensor75 of the_automated pupil monitoring system are all in communication with the automated control unit 40, such as through respective communications cables. As depicted, the automated control unit 40 includes a screen 41 upon which the eyes of the subject 50 may be monitored. The automated control unit 40 is in communication with the eyelid retractors 1 a,b and the pupilometers 30 a,b, wherein the automated control unit 40 controls the eyelid retractors and the pupilometers. For instance, the automated control unit 40 controls the retraction of the eyelid retractors 1 a,b, thereby controlling the opening and the closing of the eyes, controls the extending and retracting of the pupilometers 40 a,b, and further controls the imaging sensors or cameras and light sources of the pupilometers. The pupilometers are coupled to the surgical face mask with the imaging sensors or cameras facing the aperture.

Accordingly, in one embodiment, an automated pupil monitoring system for monitoring the pupil of a subject is provided. The system includes one or more eyelid retractors, which comprises adhesive to adhere to skin of the subject, and one or more pupilomters, that are fitted within the casing of a surgical facemask.

The face mask, therefore, includes a casing, having an interior surface and an exterior surface, at least one aperture, which communicates through the casing and is positioned in the casing to allow visualization of the subject's eyes, and a forehead rest in the interior surface of the casing, which forehead rest divides the interior of the casing into a left ocular region and a right ocular region, wherein each ocular region forms an indentation that accommodates the eyelid retractor.

The one or more pupilometers include an imaging sensor or camera and a light source that emits light, such as in the form of a flash, so as to stimulate the pupil. The imaging sensor is positioned so as to face the aperture in the casing. An automated control unit may also be provided, wherein the control unit is in communication with the eyelid retractor and the pupilometer. The automated control unit is configured for controlling the eyelid retractor and the pupilometer.

As set forth above, in certain instances, the system may further include an imaging sensor. Accordingly, in one embodiment, the pupilometer may include a pair of binocular imaging sensors and a pair of binocular light sources that can emit light in the form of a flash to stimulate the pupil. The distance between the pair of binocular imaging sensors may be adjustable.

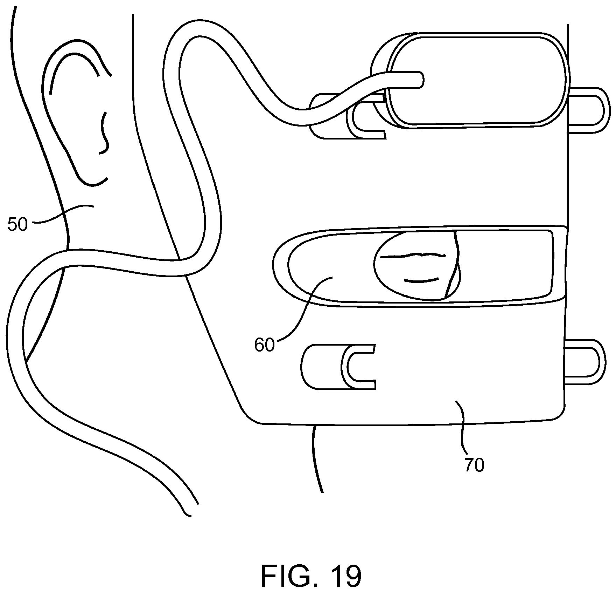

Figure 19 provides an illustration of a subject 50 wearing an ocular monitoring assembly including the surgical face mask 60 of FIG. 12, optional face mask retention casing 70 of FIG. 17, and pupilometer and imaging sensor 75 of FIG. 16.

A method of monitoring a subject's eyes during a surgical or other medical procedure or treatment is also provided. The method includes providing an eyelid retractor, such as the one shown in Figs. 1-5. The anchoring portion 10 of the eyelid retractor is anchored to the forehead of a subject 50, and the attachment member 22 is detachably attached to the eyelid 52 of the subject through its attachment interface 28b. During the procedure or treatment, the eyelid retractor 1 is used to open and close an eyelid 52 of the subject 50 to enable monitoring of the subject's pupil. During the procedure, the retraction portion 20 is retracted by pulling the connection element 24b in a proximal direction within the channel 17 in the top of the anchoring portion 10 of the eyelid retractor 1. The retraction of the retraction portion 20 pulls the attachment member 22 in the proximal direction thus opening the eyelid 52 of the subject 50. With the eyelid open, the physician or caregiver can monitor the subject's pupil. After the physician or caregiver is done monitoring the pupil, the pulling force on the connection element 24b can be released, thus allowing the retraction member to extend back to its original resting or extended position and allowing the eyelid to again close. In one embodiment, the subject can be fitted as described above with just one eyelid retractor in order to monitor one pupil of the patient, or the subject can alternatively be fitted with two eyelid retractors to monitor both pupils of the subject.

In one embodiment, the monitoring is accomplished by traditional visualization of the physician or caretaker using a light source and visualizing the pupil's response to the light source. In another embodiment, a pupilometer is used, such as the one shown in Figs. 7-10 or 16. In one embodiment, the eyelid retractor 1 is fitted with pupilometer 30. Both the eyelid retractor 1 and the pupilometer 30 are in mechanical, electrical, or wireless communication with an automated control unit 40. Housing 31b is extended with extenders 34a,b distally until the imaging sensor 32a is positioned over the calibration marker 27 on the top side of the attachment member 22. The pupilometer imaging sensor 32a is used to image the calibration marker, and data representing the image is transmitted to the automated control unit 40. This data represents a relative size of the calibration marker. In addition, data representative of the absolute size of the calibration marker 27 is contained in either a memory of the pupilometer 30 or a memory of the automated control unit 40. If it is in the memory of the pupilometer 30, it is transmitted to the automated control unit 40 in the same manner as the data representing the relative marker size. During the procedure, the eyelid is opened with the eyelid retractor 10, thus revealing the subject's pupil. The housing 31b is adjusted so that the imaging sensor is positioned over the subject's pupil. The imaging sensor has a pupil sensor that detects the boundaries of the pupil. Once detected, the imaging sensor images the pupil, and data representing the image is transmitted to the automated control unit 40. This data represents a relative size of the pupil. The automated control unit 40 contains pupil size determination software that processes data representative of the absolute size of the calibration marker 27, the relative size of the calibration marker 27, and relative size of the subject's pupil, and determines an absolute size of the subject's pupil. The light source 32b is then activated to deliver a pulse of light toward the pupil. The imaging sensor 32a is automatically synchronized with the light source 32b and captures one or more images of the pupil immediately after the pulse of light is delivered. The data representing the pupil's response to the light emitted by the light source 32b is transmitted to the automated control unit 40 for storage and processing. The retraction portion 20 of the eyelid retractor 1 is then allowed to extend again, thus allowing the subject's eyelid to close. The housing 31b of the pupilometer 30 can then be retracted toward housing 31a. The above steps can be repeated one or more times during a surgical or medical procedure or other treatment until the procedure or treatment is completed.

In one embodiment, the pupilometer 30 contains electronics, an imaging system including an imaging sensor, a light source, circuitry, and processor and software for controlling the pupilometer and storing, processing and outputting data relating to pupilometry. In another embodiment, the pupilometer 30 contains electronics, an imaging system including imaging sensor, a light source, and circuitry, but the processor and software for controlling the pupilometer and storing, processing and outputting data relating to pupilometry are contained within the automated control unit 40. In one embodiment, the electronics, imaging system, light source, circuitry, and processor and software are the same as those described in U.S. Patent No. 7,670,002. In another embodiment, the electronics, imaging sensor, light source, circuitry, and processor and software are the same as those described in U.S. Patent Application Serial No. 12/210,185, which is incorporated herein by reference. In yet another embodiment, the electronics, imaging sensor, light source, circuitry, and processor and software are the same as those described in U.S. Patent Application Serial No. 12/436,469, which is incorporated herein by reference. In yet another embodiment, the electronics, imaging sensor, light source, circuitry, and processor and software are the same as those described in U.S. Patent Application Serial No. 12/626,452, which is incorporated herein by reference. In one embodiment, the pupilometer 30 contains a separate illumination system for illuminating the pupil, such as the one described in U.S. Patent No. 7,670,002.

In one embodiment the automated control unit 40 contains a screen with a graphical user interface that displays the images obtained by the imaging sensor 32a in the pupilometer 30. In one embodiment, the automated control unit 40 contains software for controlling the pupilometer 30 and the eyelid retractor. The software can include a graphical user interface with one or more fields for accepting inputs for various control and processing parameters, such as light stimulus activation intervals from the light source 32b, amplitude of the light stimulus pulse from the light source 32b, the length of time the light source 32b is turned on during each pulse, the dynamic pupil response data that the physician or caretaker wants displayed (e.g., minimum pupil aperture, maximum pupil aperture, difference between maximum and minimum pupil apertures, latency of pupil response to stimulation, pupil constriction velocity, first and second pupil dilation velocities, and pupil irregularity magnitude and location information), and the form in which the data is displayed (e.g., graphical or numerical).

In one embodiment, the processor and software for controlling the pupilometer and storing, processing and outputting data relating to pupilometry includes an algorithm that transforms the image data and/or the dynamic pupil response data obtained by the pupilometer into a scalar value that represents a physiological condition of the subject, in which the physiological condition of the subject can be a neurological condition of the subject and/or an ocular condition of the subject. For example, the algorithm can transform one or more components of the pupil's dynamic response (e.g., minimum pupil aperture, maximum pupil aperture, difference between maximum and minimum pupil apertures, latency of pupil response to stimulation, pupil constriction velocity, first and second pupil dilation velocities, and pupil irregularity magnitude and location information) to a scalar value that represents the pathologic or neurologic condition of the subject in real-time. The scalar value can be displayed on the automated control unit 40 so that the physician or caretaker can monitor the pathologic or neurologic condition of the subject. In one embodiment, the pathologic condition is the intra-ocular pressure of one or both of the subject's eyes. In another embodiment, the scalar value represents the condition of one or both of the subject's optic nerves.

In another embodiment, a schematic illustration of an eyelid retractor 100 is shown in Figure 20. Eyelid retractor 100 includes an eyelid patch 110 that is attachable to an eyelid of a subject. Eyelid patch 110 has an adhesive backing that can be removably adhered to human skin. Adhesive backings that are adherable to human skin are known in the art. For example, 3M® makes such adhesive backings. Eyelid patch 110 is connected through a connector 115 to a movable positioner 120. The connector 115 and eyelid patch 110 can be made of a unibody construction, or can be made in two separate pieces or parts and connected to one another. The connector can be non-adhesive material while the eyelid patch contains adhesive material. Movable positioner 120 can be a metal bar or magnet. Movable positioner 120 is connected to a spring 125 on an end opposite the end to which it is connected to the connector 115. One end of spring 125 is connected to the movable positioner 120. Movable positioner 120 is set within a housing 150. The other end of spring 125 is connected to housing 150. Housing 150 forms a channel 152 in which movable positioner 120 is slidably nested. Movable positioner 120 is fixed to housing 150 only by spring 125 and is not otherwise immovably coupled to housing 150. Thus, movable positioner 120 can slide back and forth along the length of housing 150 in the direction indicated by arrows 160. Housing 150 has guide rails 154 and 156 that form lips that extend over the top of movable positioner 120 so that movable positioner 120 cannot fall out of housing 150. Alternatively, housing 150 can form a lumen rather than a channel and completely surround movable positioner 120, so that movable positioner 120 is nested within the lumen and slides back and forth within the lumen. Housing 150 also contains a magnetic coil 170. In Figure 20, magnetic coil 170 is located at the distal end of housing 150, but it can also be located at the proximal end of housing 150. Housing 150 also includes electronics 180 for providing an electric current to magnetic coil 170. Electronics 180 can include a battery and a switch to allow current from the battery to reach magnetic coil 170. Other sources of power may be remote, such as a transmitter that transmits a wireless signal to a wireless signal receiver in electronics 180, or a remote power source that delivers an electric current to magnetic coil 170 through a cable. Wireless signals can include, for example, radio-frequency signals, infra-red signals, wirelss USB signals, or Bluetooth® signals. The wireless signal can activate a switch to enable power from a local battery to reach magnetic coil 170, or can be the source of the power itself by delivering a signal that is converted to an electric current by electronics 180. Once the electric current reaches magnetic coil 170, magnetic coil 170 becomes magnetized and either attracts or repels movable positioner 120.

In one embodiment (as shown in Figure 20), magnetic coil 170 is located at the distal end of housing 150. Movable positioner 120 is proximal magnetic coil 170, and is connected to the distal end of housing 150 with a tension spring 125. The spring in its normal state keeps movable positioner 120 near magnetic coil 170. When magnetic coil 170 is magnetized, it repels movable positioner 120 causing movable positioner 120 to move away from the distal end of housing 150 and toward the proximal end of housing 150 toward the subject's eyelid. Thus, this repelling force causes the eyelid to close. When no current is applied or the current is gradually reduced, tension spring 125 pulls the movable positioner 120 back toward the distal end of housing 150, thus causing movable positioner 120 to pull against eyelid patch 110 and opening the eyelid of the subject. In an alternative, embodiment, spring 125 is a compression spring instead of a tension spring and the magnetized magnetic coil 170 and metal or magnet of movable positioner 120 attract each other instead of repel each other. Thus, during a resting and demagnetized state, the eyelid is closed because compression spring 125 pushes movable positioner 120 toward the proximal end of housing 150, i.e., toward the subject's eye, allowing the eyelid to remain closed. Once magnetic coil 170 is magnetized, movable positioner 120 is attracted to magnetic coil through an attractive magnetic force, compresses compression spring 125, and moves toward the distal end of housing 150. This movement of movable positioner 120 toward the distal end of housing 150 causes the eyelid to open, because the movable positioner pulls eyelid patch 110 in the distal direction. When no current is applied or the current is gradually reduced, the magnetic coil becomes demagnetized and the force of compression spring 125 overcomes the magnetic force and pushes movable positioner 120 proximally toward the proximal end of housing 150, thus allowing the eyelid to again close.

In yet other embodiments, magnetic coil 170 is located at the proximal end of housing 150, and the magnetic forces between the magnetized magnetic coil 150 and movable positioner 120 cause movable positioner 120 to move either toward magnetic coil 170 or away from it, thus, again causing the eyelid to open or close depending on the direction of movable positioner's 120 movement.

Eyelid retractor 100 can also include a microprocessor within housing 150. The microprocessor can include programmable memory that can be programmed to cause electronics 180 to magnetize and demagnetize magnetic coil 170. The program can have a predetermined frequency of magnetization and demagnetization. The microprocessor can also be programmed to control the amount of magnetization and demagnetization so that movable positioner 120 moves gradually and not in a jerking and sudden fashion. The importance of gradual movement of movable positioner 120 is so that the eyelid is not injured or torn by jerking sudden pulling or releasing movements. Alternatively, the programmable microprocessor that can be programmed to cause electronics 180 to magnetize and demangtize magnetic coil 170 can be contained within a remote control unit that communicates with electronics 180 through an electric cable or through a wireless signal, such as the ones described above.

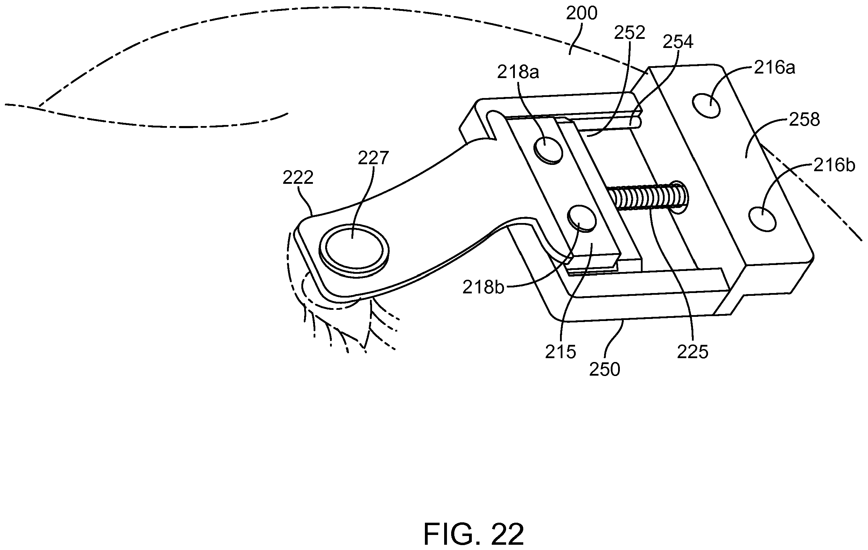

An example of one embodiment of the eyelid retractor described with respect to Figure 20 is shown in Figures 21 and 22. Figures 21 and 22 depict an eyelid retractor 200 that includes an attachment member 222 with an eyelid patch 210 on its bottom surface that is attachable to an eyelid of a subject. Eyelid patch 210 has an adhesive backing that can be removably adhered to human skin such as described above with respect to Figure 20. The top side of attachment member 222 has a calibration marker 227, which has the same function and is used in the same way as described with respect to Figures 1-11 above. Attachment member 222 is connected through a connector 215 to a movable positioner 220. The connector 215 can be connected to movable positioner 220 through screws 218a and 218b that screw the connector 215 to the movable positioner 220. Alternatively, the attachment member 222 and movable positioner 220 can be made of a unibody construction as one single part. Movable positioner 220 can be a metal bar or magnet. Movable positioner 220 is connected to a spring 225 on an end opposite the end to which it is connected to the connector 215. One end of spring 225 is connected to the movable positioner 220. Movable positioner 220 is set within a housing 250. At the distal end of housing 250 is subhousing 258. The other end of spring 225 is connected to subhousing 258. Housing 250 forms a channel 252 in which movable positioner 220 is slidably nested. Movable positioner 220 is fixed to housing 250 only by spring 225 and is not otherwise immovably coupled to housing 250, except through guide rails 254 and 256. Thus, movable positioner 220 can slide back and forth along the length of housing 250. Guide rails 254 and 256 form rails in channel 252 of mhousing 250 along which the positioner 220 slides, with the sides of the positioner forming channels that surround guide rails 254 and 256 such that the positioner 220 cannot fall out of housing 250 as a result of being secured by the rails 254 and 256.

Subhousing 258 contains a magnetic coil inside of it. Subhousing 258 also includes electronics for providing an electric current to the magnetic coil. The electronics can include a battery and a switch to allow current from the battery to reach the magnetic coil. Other sources of power may be remote, such as a transmitter that transmits a wireless signal to a wireless signal receiver in the electronics, or a remote power source that delivers an electric current to the magnetic coil through a cable communicating with the electronics through port 259. Wireless signals can include those described above with respect to Figure 20. The wireless signal can activate a switch to enable power from a local battery to reach the magnetic coil, or can be the source of the power itself by delivering a signal that is converted to an electric current by the electronics. Once the electric current reaches the magnetic coil, the magnetic coil becomes magnetized and either attracts or repels movable positioner 220. The housing 250 and subhousing 258 can be made of a non-metalic and nonconductive substance such as medical grade hard plastic.

Subhousing 258 also contains ports 216a and 216b. Ports 216a and 216b can be in electrical communication with the electronics contained within subhousing 258. Ports 216a and 216b can receive an ophthalmic instrument, such as a pupilometer 30 (as depicted in Figures 24 and 25). Ports 216a and 216b are both physical attachment ports for mounting the ophthalmic instrument 30 onto the subhousing 258 and also serve as communication ports so that the ophthalmic instrument 30 can communicate with the electronics in the subhousing 258. In this way, the electronics can be controlled by the ophthalmic instrument 30 or by a control panel that controls the ophthalmic instrument (as described with respect to Figures 1-11 above). Housing 250 can have an adhesive material on its bottom surface to adhere the housing to the forehead of a human subject. Alternateively, it can be secured to the head of a human subject with a belt or band that encircles the head.

Movable positioner 220 is proximal the magnetic coil in subhousing 258, and is connected to the proximal end of subhousing 258 as clearly shown in Figure 22 with a spring 225. In one embodiment, spring 225 is a tension spring. Tension spring 225 in its normal state keeps movable positioner 220 near the magnetic coil. When the magnetic coil is magnetized, it repels movable positioner 220 causing movable positioner 220 to move away from subhousing 258 and toward the proximal end of housing 250 toward the subject's eyelid. Thus, this repelling force causes the eyelid to close. When no current is applied or the current is gradually reduced, tension spring 225 pulls the movable positioner 220 back toward subhousing 258, thus causing the movable positioner 220 to pull attachment member 222 and opening the eyelid of the subject.

In an alternative, embodiment, spring 225 is a compression spring instead of a tension spring and the magnetized magnetic coil and metal or magnet of movable positioner 220 attract each other instead of repel each other. Thus, during a resting and demagnetized state, the eyelid is closed because compression spring 225 pushes movable positioner 220 toward the distal end of housing 250, i.e., toward the subject's eye, allowing the eyelid to remain closed. Once the magnetic coil is magnetized, movable positioner 220 is attracted to the magnetic coil through an attractive magnetic force, compresses compression spring 225, and moves toward subhousing 258. This movement of movable positioner 220 toward subhousing 258 causes the eyelid to open, because movable positioner 220 pulls attachment member 222 in the distal direction. When no current is applied or the current is gradually reduced, the magnetic coil becomes demagnetized and the force of compression spring 225 overcomes the magnetic force and pushes movable positioner 220 proximally toward the proximal end of housing 250, thus allowing the eyelid to again close.

In another embodiment, rather than a spring (as shown in Figs. 20-23), a dampener is used to cause a push force. For example, a silicon dampener can be used instead of a spring. In one embodiment, the silicon dampener can be placed between the positioner 120 and the distal end of the housing 150 (or between the positioner 220 and subhousing 258). In another embodiment, the silicon dampener can be placed between the positioner 220 (as shown in Fig. 21) and the proximal end of the housing, so that the dampener is pushing the positioner 220 toward the subhousing 258. In yet another embodiment, there is no spring or dampener, and the position of the positioner 120 or 220 is controlled entirely by the magnetic forces between the positioner and the magnetic coil 170. The position of the positioner 120 or 220 depends on the amount of magnetic force emitted by the magnetic coil 170, which is controlled by the control unit that controls the amount of electrical energy that is transmitted to the magnetic coil 170 as described above. In yet another embodiment, the position of the positioner 120 or 220 is controlled by both the force of the spring 125 or 225 or dampener and the magnetic force emitted by the magnetic coil 170, such that the magnetic force of the magnetic coil provides an additive or additional force to that caused by the spring 125 or 225 or dampener. For example, the spring 125 or 225 or dampener alone may not have enough force to keep the eyelid closed, and the magnetic force caused by the magnetic coil 170 may provide additional force to assist the spring 125 or 225 or dampener. In other words, there may be two components of force moving in the same direction: the spring 125 or 225 or dampener plus the magnetic energy caused by the magnetic coil 170, rather than the magnetic energy counteracting the spring 125 or 225 or dampener.

While the invention is susceptible to various modifications and alternative forms, specific examples thereof have been shown by way of example in the drawings and are herein described in detail.

## Claims

1. An automated ophthalmic monitoring system for monitoring an eye of a subject (50), comprising:
an eyelid retractor (1, 1a);
an ophthalmic imaging device comprising an imaging sensor (32a); and
an automated control unit (40) in communication with the eyelid retractor (1, 1a) and the ophthalmic imaging device, **characterized in that** the automated control unit controls the operation of the eyelid retractor (1) and the ophthalmic imaging device.

2. The automated ophthalmic monitoring system of claim 1, wherein the ophthalmic imaging device is a pupilometer (30).

3. The automated ophthalmic monitoring system of claim 2, wherein the pupilometer (30) comprises a light source (32b) that emits light in the form of a flash to stimulate a pupil.

4. The automated ophthalmic monitoring system of claim 2, wherein the pupilometer comprises an imaging sensor (32a) and the light source (32b) that emits light in the form of a flash to stimulate the pupil.

5. The automated ophthalmic monitoring system of claim 4, wherein the eyelid retractor (1, 1a) comprises a size calibration marker, wherein the absolute size of the calibration marker is contained in a memory of the control unit (40) or a memory of the pupilometer (30).

6. The automated ophthalmic monitoring system of claim 5, wherein the pupilometer (30) or the control unit (40) comprises pupil size determination software that processes data representative of the absolute size of the size calibration marker and the relative size of the size calibration marker and relative size of the subject's pupil obtained from the imaging sensor (32a), and determines an absolute size of the pupil.

7. The automated ophthalmic monitoring system of claim 4 further comprising:
a second eyelid retractor (1b) that comprises an anchoring portion (10b) and a retraction portion (20b); and
a second pupilometer coupled to the second eyelid retractor (1 b), the second pupilometer comprising an imaging sensor and a light emitter that can emit light in the form of a flash to stimulate the pupil, wherein the automated control unit (40) is in communication with the second eyelid retractor (1b) and the second pupilometer, and wherein the automated control unit (40) controls the second eyelid retractor (1b) and the second pupilometer.

8. The automated ophthalmic monitoring system of claim 7, wherein the automated control unit (40) comprises software that analyzes data received from the pupilometers and provides an output indicative of a relative afferent papillary defect.

9. The automated ophthalmic monitoring system of claim 7, wherein the automated control unit (40) comprises software that analyzes data received from the pupilometers and provides an output indicative of a damaged or diseased optic nerve.

10. The automated ophthalmic monitoring system of claim 4, wherein the automated control unit (40) comprises software that analyzes data received from the pupilometer and provides an output indicating the subject's neurological status.

11. The automated ophthalmic monitoring system of claim 4, wherein the automated control unit (40) comprises software that analyzes data received from the pupilometer and provides an output indicative of a neurological condition.

12. The automated ophthalmic monitoring system of claim 4, wherein the pupilometer comprises:
a first housing (31a);
a second housing (31 b) that contains the imaging sensor (32a) and the light source (32b);
at least one rod that connects the first housing (31a) to the second housing (31 b); wherein said rod slides in and out of the first housing (31a) to permit the second housing (31 b) to be manually adjusted toward or away from the first housing (31a).

13. The automated ophthalmic monitoring system of claim 4, wherein the pupilometer (30) and the automated control unit (40) communicate with one another wirelessly.

14. The automated ophthalmic monitoring system of claim 7, wherein the anchoring portion (10b) has a top side and a bottom side, the bottom side having a first attachment element for attaching the anchoring portion to a subject's forehead, wherein the retractor portion extends distally from the anchoring portion (10b) and comprises an eyelid attachment member (22b) extending distally therefrom, the eyelid attachment member (22b) having a top side and a bottom side, the bottom side having a second attachment element for attaching the eyelid attachment member (22b) to the subject's eyelid, and wherein the retractor portion pulls the attachment element in a proximal direction when the retractor portion flexes.

15. The automated ophthalmic monitoring system of claim 14, further comprising a retraction mechanism in communication with the attachment member (22b) and a retraction cable (25) that is connected on its first end to the retraction mechanism and connected on its second end to a device in the automated control unit (40) that drives retraction and extension of the cable (25).

## Patentansprüche

1. Ein automatisiertes ophthalmisches Überwachungssystem zum Überwachen eines Auges eines Subjekts (50), aufweisend:
einen Augenlid-Retraktor (1, 1a),
eine ophthalmische Abbildungsvorrichtung, die einen Abbildungssensor (32a) aufweist, und
eine automatisierte Steuereinheit (40) in Kommunikation mit dem Augenlid-Retraktor (1, 1a) und der ophthalmischen Abbildungsvorrichtung, **dadurch gekennzeichnet, dass** die automatisierte Steuereinheit den Betrieb des Augenlid-Retraktors (1) und der ophthalmischen Abbildungsvorrichtung steuert.

2. Das automatisierte ophthalmische Überwachungssystem gemäß Anspruch 1, wobei die ophthalmische Abbildungsvorrichtung ein Pupillometer (30) ist.

3. Das automatisierte ophthalmische Überwachungssystem gemäß Anspruch 2, wobei das Pupillometer (30) eine Lichtquelle (32b) aufweist, die Licht in der Form eines Blitzes emittiert, um eine Pupille zu stimulieren.

4. Das automatisierte ophthalmische Überwachungssystem gemäß Anspruch 2, wobei das Pupillometer einen Abbildungssensor (32a) und die Lichtquelle (32b), die Licht in der Form eines Blitzes emittiert, um eine Pupille zu stimulieren, aufweist.

5. Das automatisierte ophthalmische Überwachungssystem gemäß Anspruch 4, wobei der Augenlid-Retraktor (1, 1a) eine Größenkalibrierungsmarkierung aufweist, wobei die absolute Größe der Kalibrierungsmarkierung in einem Speicher der Steuereinheit (40) oder in einem Speicher des Pupillometers (30) enthalten ist.

6. Das automatisierte ophthalmische Überwachungssystem gemäß Anspruch 5, wobei das Pupillometer (30) oder die Steuereinheit (40) eine Pupillengröße-Ermittlungssoftware aufweist, die Daten verarbeitet, welche repräsentativ sind für die absolute Größe der Größenkalibrierungsmarkierung und der relativen Größe der Größenkalibrierungsmarkierung und der von dem Abbildungssensor (32a) erhaltenen relativen Größe der Pupille des Subjekts, und eine absolute Größe der Pupille ermittelt.

7. Das automatisierte ophthalmische Überwachungssystem gemäß Anspruch 4, ferner aufweisend:
einen zweiten Augenlid-Retraktor (1b), der einen Verankerungsabschnitt (10b) und einen Retraktorabschnitt (20b) aufweist, und
ein zweites Pupillometer, das mit dem zweiten Augenlid-Retraktor (1b) verbunden ist, wobei das zweite Pupillometer einen Abbildungssensor und einen Lichtemitter aufweist, der Licht in Form eines Blitzes emittieren kann, um die Pupille zu stimulieren, wobei die automatisierte Steuereinheit (40) mit dem zweiten Augenlid-Retraktor (1 b) und dem zweiten Pupillometer in Verbindung steht, und wobei die automatisierte Steuereinheit (40) den zweiten Augenlid-Retraktor (1 b) und das zweite Pupillometer steuert.

8. Das automatisierte ophthalmische Überwachungssystem gemäß Anspruch 7, wobei die automatisierte Steuereinheit (40) eine Software aufweist, welche Daten analysiert, die von den Pupillometern empfangen werden, und eine Ausgabe bereitstellt, die indikativ ist für eine relative afferente Pupillenstörung.

9. Das automatisierte ophthalmische Überwachungssystem gemäß Anspruch 7, wobei die automatisierte Steuereinheit (40) eine Software aufweist, welche Daten analysiert, die von den Pupillometern empfangen werden, und eine Ausgabe bereitstellt, die indikativ ist für einen beschädigten oder erkrankten Sehnerv.

10. Das automatisierte ophthalmische Überwachungssystem gemäß Anspruch 4, wobei die automatisierte Steuereinheit (40) eine Software aufweist, welche Daten analysiert, die von dem Pupillometer empfangen werden, und eine Ausgabe bereitstellt, die einen neurologischen Status des Subjektes anzeigt.

11. Das automatisierte ophthalmische Überwachungssystem gemäß Anspruch 4, wobei die automatisierte Steuereinheit (40) eine Software aufweist, welche Daten analysiert, die von dem Pupillometer empfangen werden, und eine Ausgabe bereitstellt, die indikativ ist für einen neurologischen Zustand.

12. Das automatisierte ophthalmische Überwachungssystem gemäß Anspruch 4, wobei das Pupillometer aufweist:
ein erstes Gehäuse (31 a),
ein zweites Gehäuse (31 b), welches den Abbildungssensor (32a) und die Lichtquelle (32b) enthält,
mindestens einen Stab, der das erste Gehäuse (31 a) mit dem zweiten Gehäuse (31 b) verbindet,
wobei der Stab in das erste Gehäuse (31 a) hinein und aus diesem heraus gleitet, um ein manuelles Einstellen des zweiten Gehäuses (31 b) zu dem ersten Gehäuse (31 a) hin oder von diesem weg zu erlauben.

13. Das automatisierte ophthalmische Überwachungssystem gemäß Anspruch 4, wobei das Pupillometer (30) und die automatisierte Steuereinheit (40) drahtlos miteinander kommunizieren.

14. Das automatisierte ophthalmische Überwachungssystem gemäß Anspruch 7, wobei der Verankerungsabschnitt (10b) eine Oberseite und eine Unterseite hat, wobei die Unterseite ein erstes Befestigungselement zum Befestigen des Verankerungsabschnitts an der Stirn eines Subjektes aufweist, wobei sich der Retraktorabschnitt distal von dem Verankerungsabschnitt (10b) erstreckt und ein Augenlid-Befestigungsteil (22b) aufweist, dass sich distal davon erstreckt, wobei das Augenlid-Befestigungsteil (22b) eine Oberseite und eine Unterseite hat, wobei die Unterseite ein zweites Befestigungselement zum Befestigen des Augenlid-Befestigungsteils (22b) an dem Augenlid eines Subjekts aufweist, und wobei der Retraktorabschnitt das Befestigungselement in proximaler Richtung zieht, wenn sich der Retraktorabschnitt beugt.

15. Das automatisierte ophthalmische Überwachungssystem gemäß Anspruch 14, ferner aufweisend einen Retraktionsmechanismus in Kommunikation mit dem Befestigungsteil (22b) und ein Retraktionskabel (25), welches an seinem ersten Ende mit dem Retraktionsmechanismus und an seinem zweiten Ende mit einer Vorrichtung in der automatisierten Steuereinheit (40) verbunden ist, die eine Retraktion und Extension des Kabels (25) steuert.

## Revendications

1. Système de surveillance ophtalmique automatisé pour surveiller un oeil d'un sujet (50), comprenant :
un écarteur de paupière (1, 1 a) ;
un dispositif d'imagerie ophtalmique comprenant un capteur d'imagerie (32a) ; et
une unité de commande automatisée (40) en communication avec l'écarteur de paupière (1, 1a) et le dispositif d'imagerie ophtalmique, **caractérisée en ce que** l'unité de commande automatisée commande le fonctionnement de l'écarteur de paupière (1) et du dispositif d'imagerie ophtalmique.

2. Système de surveillance ophtalmique automatisé selon la revendication 1, dans lequel le dispositif d'imagerie ophtalmique est un pupillomètre (30).

3. Système de surveillance ophtalmique automatisé selon la revendication 2, dans lequel le pupillomètre (30) comprend une source de lumière (32b) qui émet de la lumière sous la forme d'un flash pour stimuler une pupille.

4. Système de surveillance ophtalmique automatisé selon la revendication 2, dans lequel le pupillomètre comprend un capteur d'imagerie (32a) et la source de lumière (32b) qui émet de la lumière sous la forme d'un flash pour stimuler la pupille.

5. Système de surveillance ophtalmique automatisé selon la revendication 4, dans lequel l'écarteur de paupière (1, 1a) comprend un marqueur d'étalonnage de taille, dans lequel la taille absolue du marqueur d'étalonnage est contenue dans une mémoire de l'unité de commande (40) ou une mémoire du pupillomètre (30).

6. Système de surveillance ophtalmique automatisé selon la revendication 5, dans lequel le pupillomètre (30) ou l'unité de commande (40) comprend un logiciel de dimension de taille de pupille qui traite les données représentatives de la taille absolue du marqueur d'étalonnage de taille et de la taille relative du marqueur d'étalonnage de taille et de la taille relative de la pupille du sujet obtenue du capteur d'imagerie (32a), et détermine une taille absolue de la pupille.

7. Système de surveillance ophtalmique automatisé selon la revendication 4 comprenant en outre :
un second écarteur de paupière (1 b) qui comprend une partie d'ancrage (10b) et une partie de rétraction (20b) ; et
un second pupillomètre couplé au second écarteur de pupille (1 b), le second pupillomètre comprenant un capteur d'imagerie et un émetteur de lumière qui peut émettre de la lumière sous la forme d'un flash pour stimuler la pupille, dans lequel l'unité de commande automatisée (40) est en communication avec le second écarteur de paupière (1b) et le second pupillomètre, et dans lequel l'unité de commande automatisée (40) commande le second écarteur de paupière (1b) et le second pupillomètre.

8. Système de surveillance ophtalmique automatisé selon la revendication 7, dans lequel l'unité de commande automatisée (40) comprend un logiciel qui analyse les données reçues des pupillomètres et fournit une sortie indicative d'un défaut papillaire afférent relatif.

9. Système de surveillance ophtalmique automatisé selon la revendication 7, dans lequel l'unité de commande automatisée (40) comprend un logiciel qui analyse les données reçues des pupillomètres et fournit une sortie indicative d'un nerf optique endommagé ou malade.

10. Système de surveillance ophtalmique automatisé selon la revendication 4, dans lequel l'unité de commande automatisée (40) comprend un logiciel qui analyse les données reçues du pupillomètre et fournit une sortie indicative de l'état neurologique du sujet.

11. Système de surveillance ophtalmique automatisé selon la revendication 4, dans lequel l'unité de commande automatisée (40) comprend un logiciel qui analyse les données reçues du pupillomètre et fournit une sortie indicative d'un état neurologique.

12. Système de surveillance ophtalmique automatisé selon la revendication 4, dans lequel le pupillomètre comprend :
un premier boîtier (31 a) ;
un second boîtier (31 b) qui contient le capteur d'imagerie (32a) et la source de lumière (32b) ;
au moins une tige qui relie le premier boîtier (31 a) au second boîtier (31b) ; dans lequel ladite tige coulisse vers l'intérieur et l'extérieur du premier boîtier (31 a) pour permettre au second boîtier (31 b) d'être ajusté manuellement vers le premier boîtier (31 a) ou en s'écartant de celui-ci.

13. Système de surveillance ophtalmique automatisé selon la revendication 4, dans lequel le pupillomètre (30) et l'unité de commande automatisée (40) communiquent l'un avec l'autre sans fil.

14. Système de surveillance ophtalmique automatisé selon la revendication 7, dans lequel la partie d'ancrage (10b) possède un côté supérieur et un côté inférieur, le côté inférieur possédant un premier élément de fixation pour fixer la partie d'ancrage à un front d'un sujet, dans lequel la partie de rétraction s'étend de manière distale à partir de la partie d'ancrage (10b) et comprend un élément de fixation de paupière (22b) s'étendant de manière distale à partir de celle-ci, l'élément de fixation de paupière (22b) possédant un côté supérieur et un côté inférieur, le côté inférieur possédant un second élément de fixation pour fixer l'élément de fixation de paupière (22b) à la paupière du sujet, et dans lequel la partie de rétraction tire l'élément de fixation dans une direction proximale lorsque la partie de rétraction fléchit.

15. Système de surveillance ophtalmique automatisé selon la revendication 14, comprenant en outre un mécanisme de rétraction en communication avec l'élément de fixation (22b) et un câble de rétraction (25) qui est relié à sa première extrémité au mécanisme de rétraction et relié à sa seconde extrémité à un dispositif dans l'unité de commande automatisée (40) qui entraîne la rétraction et l'extension du câble (25).
